# EUROPEAN PATENT APPLICATION

(11) **EP 1 626 085 A1**
(43) Date of publication of application: **15.02.2006**
(21) Application number: 05254974.8
(22) Date of filing: 10.08.2005
(51) Int. Cl.: C12N 15/00, C12N 15/10

(54) **Devices and methods for isolating RNA**

(30) Priority: 10.08.2004 US 914920; 11.11.2004 US 985704
(71) Applicant: Agilent Technologies, Inc., Palo Alto, CA 94306 (US)
(72) Inventor: Hall, Gerald Edward, Morrisville, PA 19087 (US); Boves, Barry E., Wilmington, DE 18808 (US)
(74) Representative: Tollett, Ian

(57) **Abstract**

Devices and methods for isolating nucleic acid are disclosed herein. In particular, the isolation of total cellular RNA is discussed. Additionally, devices and methods for reducing genomic DNA in a biological sample without introducing harmful contaminants, and without significantly increasing the time required for the overall procedure being performed on a sample are presented.

## Description

The present invention relates to a method of preparing an RNA sample from a tissue and to a kit for isolating RNA.

Many molecular biological techniques, including gene expression techniques such as hybridization arrays, reverse transcription polymerase chain reaction (RT-PCR), cloning, restriction analysis, and sequencing require the input of high-purity, intact RNA. The RNA should be substantially free of contaminants that can interfere with the procedures. Such possible contaminants include substances that block or inhibit chemical reactions such as nucleic acid or protein hybridizations, enzymatically catalyzed reactions and other reactions used in molecular biology, substances that catalyze the degradation or de-polymerization of a nucleic acid or other biological material of interest, or substances that provide "background" indicative of the presence in a sample of a quantity of a biological target material (such as nucleic acid) when the nucleic acid is not actually present in the sample in question. Other contaminants can include enzymes, other types of proteins, polysaccharides, or polynucleotides, as well as lower molecular weight substances such as lipids, low molecular weight enzyme inhibitors, or oligonucleotides. Contamination can also be introduced from chemicals or other materials used to isolate the material in question. Contaminants of this type can include trace metals, dyes and organic solvents. In addition, isolation of nucleic acids is complicated by the complex systems in which the nucleic acids are typically found - including tissues, body fluids, cells in culture, agarose or polyacrylamide gels or solutions in which target nucleic acid amplification has been carried out.

Thus, the preparation of such high purity, intact RNA that can be used in a subsequent molecular biological technique often involves tedious multi-step processes, and conventional nucleic acid isolation procedures have significant drawbacks. Such drawbacks include the fact that many of the current methods include steps of organic extraction which involve the use of toxic chemicals such as phenol (a known carcinogen), volatile reagents such as chloroform (which is highly volatile, toxic and flammable) and are difficult to perform in an automated or high throughput fashion. Additionally, use of organic solvent extraction methods results in organic wastes that must be disposed of in a regulated and environmentally conscientious manner. Another drawback is the time required for the multiple extraction steps needed to isolate a given nucleic acid material. Thus, even under ideal circumstances and conditions, most conventional nucleic acid isolation methods are time-consuming, hazardous, and end up producing relatively low yields of isolated nucleic acid material.

Some commercially available isolation systems developed as an alternative to, or in addition to, the conventional isolation techniques mentioned above often involve the use of a silicate or glass-fiber filter as a nucleic acid binding substrate. It is well known that nucleic acids will bind to silicon-containing materials such as glass slurries and diatomaceous earth. The difficulties with some of these materials is that the required silicate material is often not readily commercially available in the appropriate form, and often must be prepared on-site which adds additional time and effort to the nucleic acid isolation procedure.

Silica-based systems and methods have also been developed in recent years for use in isolating total RNA from at least some types of biological materials. The known silica-based RNA isolation techniques employ the same basic sequence of steps to isolate target RNA from any given biological material. However, the concentrations and amounts of the various solutions used in each procedure vary depending on the composition of the silica-based material used. In general, the basic sequence of steps used in all known silica-based RNA isolation processes consists of: disruption of the biological material in the presence of a lysis buffer; formation of a complex of nucleic acid(s) and a "silica-based matrix"; removal of the lysis buffer mixture from the resulting complex and washing of the complex; and elution of the target nucleic acid from the complex. In general, the term "silica-based" is used to describe SiO₂ compounds and related hydrated oxides.

In recent years there have also been developed methods for purification of DNA and RNA that involve binding the nucleic acid to silicon carbide particles and then eluting the nucleic acid from the silicon carbide. Note that silicon carbide is not "silica-based" as defined above and would not be included in any composition of matter that is defined as being "silica-based".

While the various kits available provide a relatively rapid means of isolating DNA or RNA from a variety of biological materials, to those skilled in the art, however, there are known limitations in the use of silica-based nucleic acid isolation kits, as specifically applied to the isolation of RNA from biological sources. With certain complex biological samples purity of RNA can be poor, and recovery of intact RNA can be poor, especially when processing samples from a small number of cells, or when isolating RNA from certain challenging mammalian tissues, such as the pancreas, the spleen, or lung tissues.

Genomic DNA (gDNA) is a common contaminant of RNA isolations. Some commercially available RNA isolation kits provide a protocol for selective enzymatic removal of contaminating gDNA with Deoxyribonuclease I (DNase I). Treatment with DNase I occasionally results in a reduction of RNA yield and degradation of RNA by ribonucleases (RNases) that can contaminate commercially produced DNase I. DNase I treatment adds hands-on time, extends the length of time required for the process, and requires the addition of metal ions which can interfere with downstream processes.

Therefore it is desirable to have an easy, rapid, safe method and device for removing gDNA from a sample, which does not add any significant time to the overall procedures being performed, does not produce dangerous wastes that require special disposal, and which produces isolated RNA that is substantially free of contaminants including proteins, lipids, gDNA, and any chemicals likely to inhibit or interfere with further processing or analysis of the isolated RNA. In addition, it is desirable to have an easy, rapid, safe and effective method for concentrating and isolating nucleic acids on a non-silica-based material but which provides better yields than the currently available non-silica-based methods.

The present invention pertains to devices and methods for isolating nucleic acids. In particular, the invention is directed toward the isolation of total cellular RNA ("tcRNA"). Additionally, the invention relates to devices and methods for reducing gDNA in a biological sample without introducing harmful contaminants, and without significantly increasing the time required for the overall procedure being performed on a sample.

In one embodiment, the removal of a substantial amount of gDNA while maintaining RNA integrity in a sample is disclosed. This embodiment includes the use of a pre-filtration column that is packed with at least one layer of glass fibers or borosilicate fibers and in one aspect, preparing a tissue/cell lysate preparation. The preparation is then introduced into the pre-filtration column. During passage of homogenate through the pre-filtration column, cellular contaminants, including gDNA, remain within the column while the effluent contains partially purified tcRNA. In one aspect, the use of the pre-filtration column can be used prior to subjecting the sample to further purification or downstream processes.

In another embodiment, a method for isolating nucleic acids from a complex sample matrix is disclosed. In a particular aspect of this invention, the nucleic acid is RNA. This method involves disrupting the sample matrix using a chaotropic agent. Organic solvents can now be added to the samples in order to optimize subsequent processes, including tcRNA isolation. This preparation can then be introduced into a column of the present invention, for example, a silicon carbide column. In a particular aspect, the column is a silicon carbide whisker column ("SiCw"). Effluent will pass through the column and subsequent washes can assist in the elimination of contaminants from the column. Finally, the desired isolated nucleic acid product can be eluted from the column.

In another embodiment, nucleic acid is isolated using a pre-filtration column in conjunction with an isolation column like a SiCw or a "silica-based" column. In this embodiment, a tissue/cell lysate is prepared and subjected to pre-filitration. This pre-filtration step includes the use of a pre-filtration spin column. Examples of a pre-filtration spin column include, but are not limited to, a glass fiber column or a borosilicate column. In one aspect of this embodiment, gDNA remains within the pre-filtration column substrate while RNA flows through. Additionally, RNA in the effluent can be further treated with a DNase to degrade any DNA that might have not been completely removed during the pre-filtration step. The RNA-containing effluent can be subjected to an isolation column. The isolation column is employed to purify RNA from the effluent. Optionally, gDNA can be removed by DNase treatment of the RNA whilst captured within the isolation column. In any case, the RNA can finally be subsequently eluted in a small volume.

In yet another embodiment, the present invention pertains to a device that comprises silicon carbide. In a particular aspect, a SiCw column is employed as a nucleic acid binding column used to isolate nucleic acids from a sample matrix. In one aspect, the SiCw binds RNA.

In one embodiment of the present invention, an RNA isolation membrane column comprising an inlet and an outlet between which lies a chamber is disclosed. Within the chamber is a single or multiple layers of a polymeric membrane, examples of which are polysulfone, PVDF, BTS, nylon, nitrocellulose, PVP (poly(vinyl-pyrrolidone)) and composites thereof. This RNA isolation column further comprises a retainer ring and a frit, which are both disposed about the membrane. The retainer ring is disposed proximal to the inlet, while the frit is disposed proximal to the outlet.

Methods for isolating RNA using the RNA isolation membrane column are also disclosed herein. A sample matrix is prepared. The sample matrix can comprise animal, plant tissue and cells. Tissue and/or cells can be obtained from an organism using methods well known to those skilled in the art. The preparation can be subjected to lysis, exposing and/or releasing the internal components of tissues and cells. In one aspect, the lysis preparation can be subjected to a prefiltration column. Preferably, the column should be centrifuged or have a vacuum applied to it to pass the lysate through the glass-fiber prefilter column; no "layers" are formed during this centrifugation (i.e., no pellets, or phase separations). Following centrifugation, an alcohol can be added to the preparation. The alcohol-containing preparations can then be loaded on to an RNA membrane isolation spin column of the present invention. The column comprises at least one polymeric membrane of, e.g., MMM membrane. Once loaded, the columns are subjected to centrifugation. The flow through is disposed of and the spin columns are washed at least once. The RNA originated from the original sample matrix can then be eluted using nuclease-free water, or other benign low ionic strength solutions.

Another embodiment of the present invention is directed to a method of purifying cRNA using a spin column of the instant invention. In one aspect of this embodiment, the cRNA is labeled using, for example, a dye molecule such as cyanine, though other labels well known to those skilled in the art can be employed as well. The column of the present embodiment comprises a membrane that captures the labeled cRNA molecule. Impurities from the labeling reaction, and elsewhere, can be washed away subsequent to the cRNA capture using appropriate buffers.

A kit is also an embodiment of the present invention. The kit of the present invention comprises at least one pre-filtration column. In one aspect, the pre-filtration column is constructed using a fiber material, such as glass or borosilicate fibers. The kit also comprises at least one RNA isolation membrane column. Reagents are also part of the kit of the present embodiment. The reagents include at least one organic solvent and at least one lysis buffer (such as described herein). Other reagents can be included with in the kit. Such reagents could include those used for washing the membrane-isolated RNA, to further effect removal of contaminants, or to reduce the concentration of lysis reagents carried over during the collection of the tcRNA. Instructions for a practitioner to practice the invention is also included.

The present embodiment is directed to compositions and methods used to isolate total RNA from plants. The methods described herein are easy, rapid, qualitative and quantitative methods for isolating plant RNA virtually free of gDNA. The present methods employ an aqueous buffer comprising guanidine hydrochloride in conjunction with at least one spin column. In one aspect, the method includes two spin columns; a pre-filter that removes contaminating gDNA, cell debris and other contaminants followed by an RNA filter that captures the purified RNA. Typically, RNA yields are equal to or greater than silica based aqueous extraction methods and absorbance ratios at 260/280 and 260/230 are well above 1.8, indicating that the samples are free of protein and polysaccharide contamination, respectively. DNA contamination of RNA isolated with the pre-filter is around 1000 to 10,000 fold lower than samples isolated without the pre-filter.

A further embodiment is directed to kit for plant RNA isolation. The present kit comprises an aqueous buffer. This buffer comprises guanidine hydrochloride (~5.5M), Bis-Tris (~50mM, pH ~ 6.6), EDTA (~10mM) and around 1% β-mercaptoethanol. A wash solution is also included wherein the wash comprises about 70% ethanol, Tris(pH ~8), and EDTA. The kit further comprises at least one spin column of the present invention. In one aspect, the kit also includes a pre-filter of the present invention.

In another embodiment, present invention pertains to the methods for collecting nucleic acids using polyethylene glycol (PEG). In one aspect, the nucleic acid that is collected is RNA. In certain aspects, methods of the present invention include the use of PEG in conjunction with one or more spin columns described herein for the isolation of nucleic acids. In one aspect, the tissue sample originates from samples high in waxes, latex, oils and/or resins, for example, the tissue may be from a plant. In another aspect of the present invention, prefiltration to remove undesired components from the sample may performed. For example, contaminating cell debris and/or gDNA may be removed.

A number of preferred embodiments of the present invention will now be described with reference to the drawings, in which:
FIG. 1 is a schematic of the SiCw column according to one aspect of the present invention;
FIG. 2 is a schematic of the pre-filter column according to one aspect of the present invention;
FIG. 3 is an illustration of an RNA isolation column;
FIG. 4 show the results obtained following a denaturing agarose gel of RNA isolated from pancreas;
FIG. 5 is a plot showing the quantitative RT-PCR comparison between standard RNA using methods according to embodiments of the invention and RNA from Qiagen;
FIG. 6 is a plot showing the quantitative RT-PCR comparison between standard RNA using the methods according to embodiments of the invention and RNA from Qiagen with on-column DNase;
FIG. 7 is a plot showing the quantitative RT-PCR comparison between standard RNAs using the methods according to embodiments of the invention with on-column DNase;
FIG. 8 is a bar graph showing RNA mass recoveries from various spin column membrane formats;
FIG. 9 shows results obtained from an RNA isolation procedure using a variety of mammalian tissues and cell cultures and employing a variety of glass fiber type filters according to embodiments of the invention, varying the number and type of layers;
FIG. 10 shows the results obtained from the RNA isolation from plant tissues;
FIG. 11 demonstrates the results of RNA isolation from plant tissue;
FIG. 12 is a BioAnalyzer image of isolated RNA;
FIG. 13 is a spin column of the present invention;
FIG. 14 is a graphical representation of data obtained from using various columns to purify cRNA;
FIG. 15 is an agarose gel containing cRNA prepared using various columns;
FIG. 16 is an agarose gel demonstrating the wide range of species and tissue types that can be subjected to the methods according to embodiments of the invention for isolating RNA;
FIG. 17 is an agarose gel comparing plant RNA isolation employing known isolation schemes with methods according to embodiments of the invention described herein;
FIG. 18(a) depicts the results of real-time PCR to detect gDNA, and (b) depicts the visualization of gDNA contamination;
FIG. 19 depicts the effect of various compounds on RNA isolation from White Pine needles, RNA was isolated from 40 mg of White Pine Needles, wherein (a) is a graph of the total nucleic acid yield, (b) represents gDNA contamination, (c) represents RNA purity using the absorbance ratio A₂₆₀/A₂₈₀, and (d) represents RNA purity using absorbance ratio A₂₆₀/A₂₃₀;
FIG. 20 is a scan of RNA isolated from various species high in resin or latex with and without PEG3000; and
FIG. 21 illustrates the effect of increasing concentrations of PEG on total RNA isolation where (a) depicts total nuclec acid yield, (b) depicts gDNA contamination, (c) depicts RNA purity as a function of absorbance ratio A₂₆₀/A₂₈₀, and (d) depicts RNA purity as function of absorbance ratio A₂₆₀/A₂₃₀.

The present invention pertains to devices and methods used for isolating nucleic acids. In particular, the invention is directed toward the isolation of tcRNA. In a related manner, the instant invention relates to devices and methods for reducing gDNA in a biological sample without introducing harmful contaminants, and without significantly increasing the time required for the overall procedure being performed on a sample.

The present invention pertains to methods for nucleic acid isolation from a complex sample matrix. In a particular aspect of this invention the nucleic acid is RNA. In a further aspect, the RNA is tcRNA. The biological sample of the present invention includes, but is not limited to, cells and tissue obtained from eukaryotic and prokaryotic sources, such as animals, plants and bacteria. In one aspect, the animal can be a mammal, and in a further aspect, the mammal can be a human. Additional samples are envisaged, for example, plants, yeast, fungi, and virus. In addition, the sample can originate from experimental protocols, for example, from a polymerase chain reaction or the product from enzymatic polymerization, nucleic acids present in a medium such as an agarose gel or alike. The sample matrix may be comprised of single-stranded or double-stranded nucleic acids, like single or double-stranded RNA and single or double stranded DNA. Partially single-stranded nucleic acids are also included within the scope of the invention. Modified nucleic acids and nucleic acid analogs are also encompassed and are within the scope of the present invention.

Prefiltration methods and devices of the present invention not only remove gDNA contamination, but also simultaneously homogenize the sample. This simultaneous gDNA removal and homogenization is especially advantageous with those samples with which lysis and homogenization are not normally completed in a single step, such as with power homogenization. For example, cell cultures typically are lysed in a cell culture vessel or tube employing lysis solutions well known to those skilled in the art. Failure to subsequently homogenize the lysed cell culture sample can result in increased sample viscosity and reduced or variable RNA yields. Therefore, the simultaneous lysis and homogenization provided by the present invention can eliminate the need for a separate homogenization step and the attendant problems if that homogenization step is not performed.

The methods of the instant invention result in intact and highly purified total cellular RNA (tcRNA). Purified tcRNA can be defined as that from which contaminants from the sample matrix and contaminants from the process are essentially completely removed. These contaminants include chaotropic and non-chaotropic salts, alcohols, gDNA, proteins, lipids, carbohydrates as well as other cellular debris. Assays to detect contaminants include, but are not limited to, electrophoretic and spectrophotometric methods and functional assays such as PCR or reverse transcription.

Generally, the first step in the isolation of nucleic acid is the disruption of the sample and lysing the cells contained therein, using methods well known to those skilled in the art. In one aspect of the present invention, the nucleic acid to be isolated is tcRNA. An example of methods to produce high purity, intact RNA include methods described in Chomczynski, P., Sacci, N., Single-step Method of RNA Isolation by Acid Guanidinium Thiocyanate-Phenol-Chloroform Extraction. Anal.Biochem. 1987 Apr; 162(1):156-9; and US Patent No. 4,843,155 to Chomczynski, the entire teaching of which is incorporated herein by reference. Additional methods include Chapter 2 (DNA) and Chapter 4 (RNA) of F. Ausubel et al., eds., Current Protocols in Molecular Biology, Wiley-Interscience, New York (1993), the entire teaching of which is incorporated herein by reference.

Examples of lysis and organic extraction methods for isolating total RNA from various types of biological materials are found in Molecular Cloning by Sambrook, et al., 2nd edition, Cold Spring Harbor Laboratory Press, P. 7.3 et seq. (1989); Protocols and Applications Guide produced by Promega Corporation 3rd edition, p. 93 et seq. (1996); and by Chirgwin J.M. et al., 18 Biochemistry 5294(1979); and for a review of conventional techniques as well as silica-based techniques see US Patent No. 6,218,531, the entire teaching of which is incorporated herein by reference.

The present method includes use of one or more chaotropic salts. The chaotrope used can be, for example, guanidine, ammonium isothiocyante, or guanidine hydrochloride. One skilled in the art will appreciate that other chaotropes can be used and remain within the scope of this invention. Typically, the concentration of the chaotrope ranges from about 0.5 M to about 5.0 M. Again, these concentrations can vary depending upon the sample matrix as well as other factors known to those skilled in the art. Chaotropic agents are used, for example, to denature proteins and to inhibit intermolecular interactions, and importantly to inhibit the action of nucleases that can be present and may degrade the nucleic acid of interest. Monitoring nucleic acid integrity throughout the process can be performed by several methods, most commonly by electrophoretic methods and by RT-PCR assays.

A homogenate is formed upon disruption of the sample matrix and lysis of the cells contained therein by methods well known to those skilled in the art. This homogenate can be processed further.

Examples of further processing include US Patent No.'s 6,177,278 and 6,291,248 to Haj-Ahmad that describe the use of silicon carbide particles for nucleic acid isolation, all of which are incorporated herein in their entirety by reference. The nucleic acid isolation articulated by Haj-Ahmad involves the use of silicon carbide grit, a nonporous, irregularly shaped collection of particles of a relatively low specific surface area (m2/g).

As an alternative to silicon carbide, silica materials such as glass particles, glass powder, silica particles, glass microfibers, diatomaceous earth, and mixtures of these compounds are employed in combination with aqueous solutions of chaotropic salts to isolate nucleic acids.

In one aspect, the methods involve subjecting the lysis preparation to a prefiltration spin column resulting in a clarified homogenate. The lysis solution preferably contains a chaotropic salt and/or additives to protect the target nucleic acid from degradation or reduced yield. In one aspect, the prefiltration column is a glass fiber or borosilicate fiber column. In one aspect, the fiber of the present invention is binder-free. An example of a binder-free fiber is "pure borosilicate." In another aspect, the fiber employed can comprise a binder. Binders can improve handling the solid-phase filtration material. Binders may also be present resulting from a process employed to modify the characteristics of a composite material. Such process elements should be selected by compatibility with optimum yield and purity of the target nucleic acid. Examples of such binders include, but are not limited to, acrylic, acrylic-like, or plastic-like substances. Although it can vary, typically binders represent 5% by weight of the fiber filter.

Another aspect of the present invention involves the addition of an organic solvent. For example, a low molecular weight alcohol such as ethanol, methanol or isopropanol in the range of about 50-80% by volume. The organic solvent improves the purity, and/or permits the high recovery of the target nucleic acid.

In the prefiltration step, gDNA along with other contaminants remain within the spin column and the effluent will contain the desired RNA. Optionally, this effluent can be treated with a DNase to degrade any DNA that escaped the column ending up in the effluent.

In one aspect, the filter fiber material demonstrates particle retention in the range of about 0.1 µm to about 10 µm diameter equivalent. The fiber can have a thickness ranging from about 50 µm to about 2,000 µm. For example, a typical fiber filter has a thickness of about 500 µm total thickness. The specific weight of a fiber filter typically ranges from about 75 g/m2 up to about 300 g/m2. Multiple fiber layers are envisaged to be within the scope of this invention.

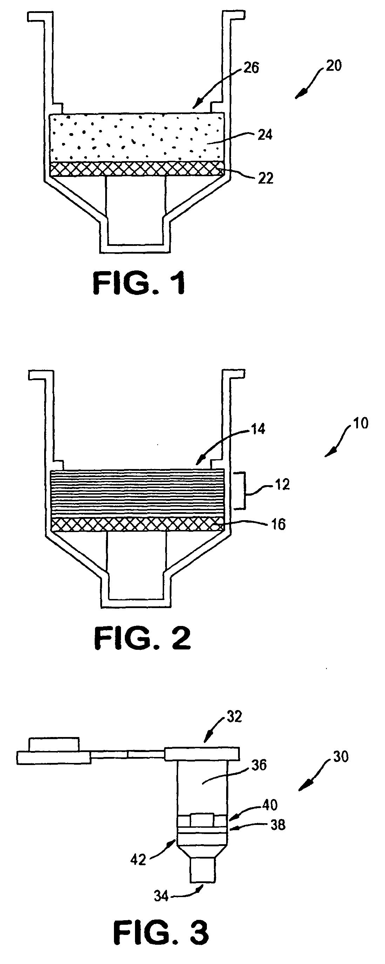

An example of a typical SiCw column of the present invention is shown in Figure 1. Shown in this figure is a spin column 20, having a frit 22 placed therein. Atop of this frit 22, silicon carbide whiskers 24 are introduced. A retainer ring 26 is placed adjacent to the bed of silicon carbide whiskers 24 in order to secure the material and prevent the silicon carbide whiskers from swelling excessively.

This silicon carbide whisker has a comparatively high specific surface area material for nucleic acid isolation. The SiCw used here are 3.9 m2/g as measured by surface Nitrogen absorption. The whisker technology performs effectively for nucleic acid, particularly RNA, isolation from complex samples. The present invention results in intact RNA and provides a method for removal of gDNA.

As previously mentioned, once the SiCw spin column has been loaded with sample, it can then be placed in a collection tube to be centrifuged, or placed on a vacuum manifold. The spin column is then centrifuged in a micro-centrifuge, or a vacuum is applied to the manifold, and the sample preparation passes through the SiCw filter in the spin column and into a collection chamber. Most of the nucleic acid, *i.e.*, RNA and any gDNA not removed by the prefiltration process, remains within the SiCw spin column. See Tables 5 and 6 below in the Examples section.

Optionally, subsequent steps following the addition of the sample preparation to the spin column can include washing and optional enzymatic treatments to remove contaminants. For example, such treatment can include the use of DNase, (DNase I or II). DNase treatment subsequent to sample binding will remove residual gDNA contamination, however, such treatment may not be necessary. While DNase I is the most commonly used DNase, DNase II could also be used. DNase II is isolated from spleen and has slightly different properties such as in its apparent molecular weight, optimal pH, and perhaps recognizes and cuts at different bases than DNase I which is isolated from pancreas. However, both enzymes are commercially available, in an RNase-depleted form, which is critical to ensure intact RNA following DNase digestion. If DNase treatment is used, after the homogenate is passed through the column, the column can be washed, for example, once with Wash Buffer #1 comprising a chaotropic salt, such as guanidine isothiocyanate, ammonium isothiocyanate and guanidine hydrochloride, at a concentration of at least 0.5 M and about 5% and up to about 10% of a low molecular weight alcohol such as methanol, ethanol, isopropanol, or alike and is buffered to a pH in the range of about pH 6 to about pH 9. For example, the RNase-free DNase in a buffered solution (pH between 6 and 9) containing calcium chloride and magnesium chloride (or sulfate or manganese chloride) is applied to the sample-bound substrate and allowed to incubate for at least 5 minutes in temperatures ranging from about 25°C to about 37°C.

After this incubation, Wash Buffer #1 is applied to the homogenate in the column. The column is then centrifuged and/or a vacuum is applied to the column.

Following the wash with Wash Buffer #1, two subsequent washes can be performed using Wash Buffer #2 which comprises 25 mM Tris-HCl, pH 7 (Ambion, Austin, TX) and 70% ethanol (Sigma, St. Louis, MO). Typically, Wash Buffer #2 contains about 50% to about 80% of a low molecular weight alcohol, *e.g.*, ethanol, methanol, isopropanol or alike, by volume.

As described supra, the washing solutions are removed either by centrifugation and/or vacuum removal. The centrifugation and/or vacuum procedures remove the majority of the alcohol from the column material.

If DNase digestion is not performed, after passing the sample through a filtration column, the column is washed at least once with Wash Buffer #1 before washing with Wash Buffer #2. Following the washes, the column is eluted. For example, if a SiCw column is used, the final step is the elution of the isolated, purified nucleic acid, *e.g.*, tcRNA, from the SiCw column. Solutions used to elute the SiCw column have generally low ionic strength, less than 100 mM, with a pH ranging from about 6.0 to about 8.5. Two examples of such solutions are 10 mM EDTA and 10 mM sodium citrate.

Additionally, a second round of isolation can occur. DNase digestion can be performed on the total elution from the SiCw (or other binding) column. Purification, including the washing steps, can then be done using a different column. When the DNase digestion is performed after elution, it can be done in the same collection tube, using the entire sample, or an aliquot can be removed. Typically the DNase reaction performed after elution uses fewer units of the DNase enzyme under similar buffer conditions. The post-elution DNase digestion can be done at 37°C for a shorter period of time than the 15 minutes used in the DNase digestion prior to elution. The reaction can then be terminated with EDTA, and the enzyme heat inactivated at, for example, 65°C, and/or subjected to additional cleanup procedures potentially including phenol/chloroform extractions or alike.

Figure 2 depicts a typical embodiment of a pre-filtration spin column 10 of the present invention. In a particular aspect of this invention, the pre-filtration column comprises at least one layer (in this figure, multiple layers) of fiber filter material 12 along with a retainer ring 14 that is disposed adjacent to a first surface of the fiber filter material which securely retain the layers of fiber filter material 12 so that they do not excessively swell when sample is added. Also depicted is a frit 16 that is disposed adjacent to a second surface of the fiber filter material 12. In one aspect, the frit 16 is composed of polyethylene of about 90 µm thick. The frit 16 assists in providing support so that the materials of the filter fibers 12 do not deform.

In a further aspect of the present invention, the effluent preparation is subjected to further purification using a filtration column such as a silicon carbide column, for example, the silicon carbide whisker column of the present invention (FIG. 1). Once a sample has been pre-filtered on the fiber filter of the present invention, it can be used in any subsequent procedure. The SiCw column with the homogenate disposed therein can then be placed in a collection tube in a centrifuge unit to be centrifuged and/or placed on a vacuum manifold. The silicon carbide whisker column can then be centrifuged in a micro-centrifuge (~2 min. at 16,000 x g) and/or a vacuum can be applied to the manifold, and the effluent passes through the SiCw filter into a collection chamber. Most of the target nucleic acid of interest remains bound to the silicon carbide whiskers. In a particular aspect, the nucleic acid of interest is RNA. And in a more particular aspect, the RNA is tcRNA.

The present invention also includes pre-packaged or individually available components of a kit for RNA isolation using the methods and devices of the present invention. A typical kit can include: packed fiber pre-filters with collection tubes; packed SiCw spin columns with collection tubes or a slurry of SiCw with plastic-ware for a practitioner to pack the spin column(s), or dry SiCw for the practitioner to slurry and pack; reagents including Wash Buffers #1 and #2, an alcohol such as ethanol and β-metcaptoethanol; and collection tubes for elution. Kits can also be prepared and assembled with DNase and/or Proteinase K, and the components comprising the kit can be obtained separately as well.

In one embodiment of the present invention, an RNA isolation column 30 comprising an inlet 32 and an outlet 34 between which lies a chamber 36 is disclosed. Within the chamber is a single or multiple layers of a polymeric membrane 38, examples of which include polysulfone, PVP (Poly(vinylpyrrolidone)), MMM membrane (Pall Life Science), BTS, PVDF, nylon, nitrocellulose, and composites thereof. The membrane need not be polymeric. A retainer ring 40 an a frit 42 are disposed about the membrane38. The retainer ring 40 is disposed proximal to the inlet 32, while the frit 42 is disposed proximal to the outlet 34.

An important aspect of the present invention is that polymeric membranes do not suffer from the limitations of silica-based columns such as solubility at elevated pH or irreversible absorption.

Using the RNA isolation membrane column 30 of one embodiment of the present invention, a practitioner can add a sample between 5 - 700 µL per "loading" or "spinning" to the column 30 via the inlet 32. Prior to adding the sample to the column 30 of the present invention, the sample matrix can then be disrupted using, for example, a chaotropic agent and subjected to centrifugation. If desired, the practitioner can add one or more organic solvents to the preparation. For example, between 0.25 and 1 volume of 25-100% alcohol can be added and subjected to centrifugation. The membrane collects the precipitate and the "flow-through" that is collected in a collection tube during centrifugation can be readily disposed. Following these preliminary steps, the sample preparation can then be added to the RNA isolation membrane column 30. Effluent will pass through the column and subsequent washes using wash buffer #2 will facilitate the elimination of contaminants from the column. Through this process, the desired RNA will have precipitated out along one or more membrane 38 surfaces. The RNA can now be harvested using methods well known to those skilled in the art.

Optionally, the sample matrix can be subjected to a fiber prefiltration column (such as glass or borosilicate) prior to the addition of organic solvent, this can aid in the reduction of gDNA contamination compared to the methods of commercially available silica-based kits (see US patent application 10/631,189, the entire teaching of which is incorporated herein by reference). Further reduction of gDNA can be accomplished via an on-column DNase digestion. This digestion may be considered optional and is not necessary for reduction of gDNA. Quantitation of contaminating gDNA is accomplished through a direct quantitative PCR assay.

The advantages of this method include, but are not limited to, reduced sample preparation time due to fewer steps, reduced elution volume resulting in concentrated samples, and reduced genomic DNA (gDNA) contamination due to the pre-emptive removal of gDNA via, for example, using a glass-fiber prefiltration column. There are no toxic or caustic substances such as phenol or chloroform that are required in the RNA isolations of the present method, however, should a practitioner elect to employ such substances, one may do so and still be within the scope of the present invention.

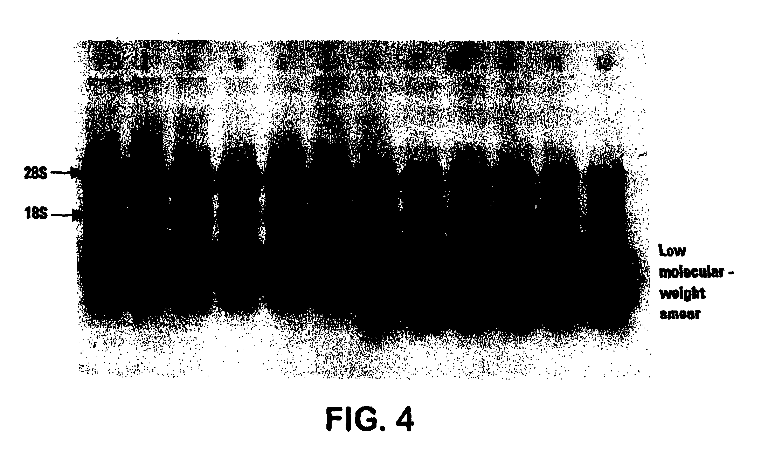

Comparison of RNAs isolated using the present methods with those from using commercial methods, such as QIAGEN RNeasy Mini Kit (QIAGEN, Valencia, CA, part no. 74104) with and without optional on-column DNase digestion are shown in Tables 1-4. The data demonstrates that RNA isolated using the columns and methods of the present invention is of sufficient and equivalent quantity while also demonstrating reduced gDNA contamination. Furthermore, with difficult samples the present invention can result in RNA of increased quality. A denaturing agarose gel demonstrating this is in FIG. 4. RNAs isolated from pancreas using the present method or QIAGEN RNeasy Mini Kit with and without optional on-column DNase digestion are shown in FIG. 4. Lanes 1-3 are standards from the present method, 4-6 is the on-column DNase treated using the methods disclosed herein, 7-9 are Qiagen's standard, 10-12 are Qiagen's on-column DNase treated samples. Low molecular-weight smears in the lanes containing RNA resulting form the QIAGEN methods are indicative of degradation. In the lanes containing RNA resulting from the present method, there are no low molecular-weight smears and the ribosomal RNA bands are in the characteristic 28S:18S ratio of 2:1, respectively.

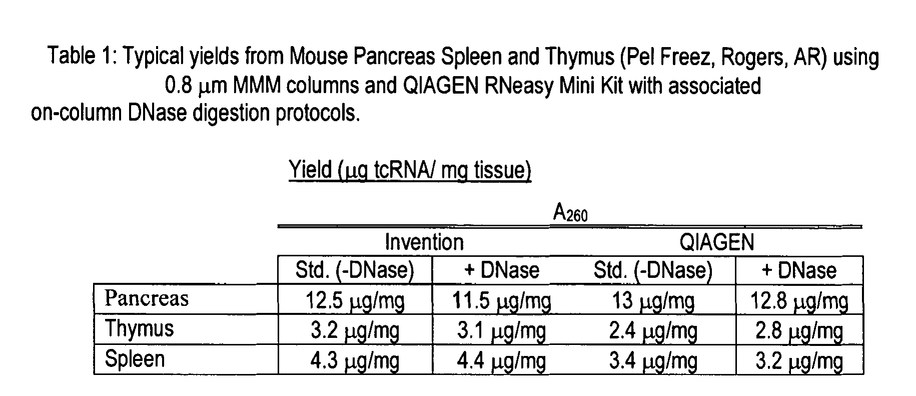

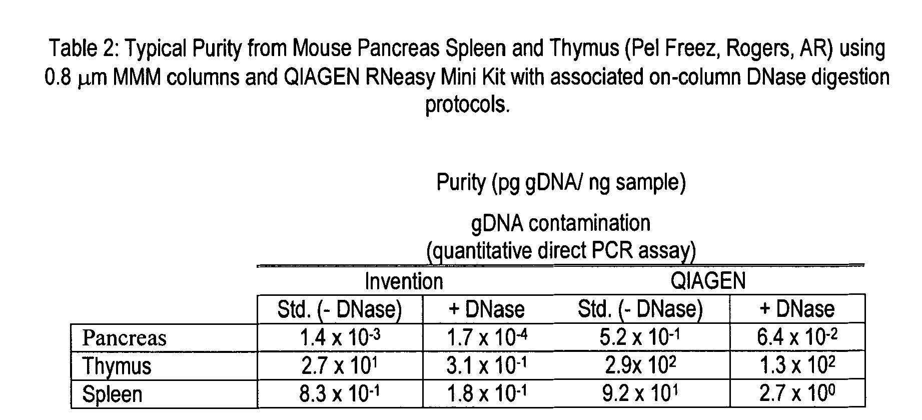

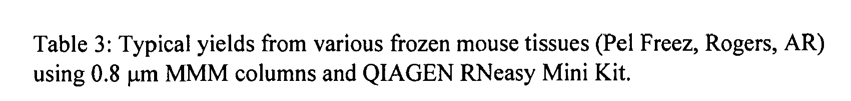

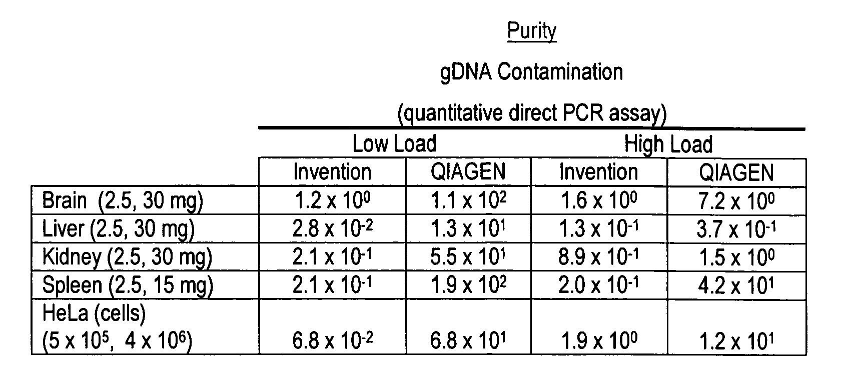

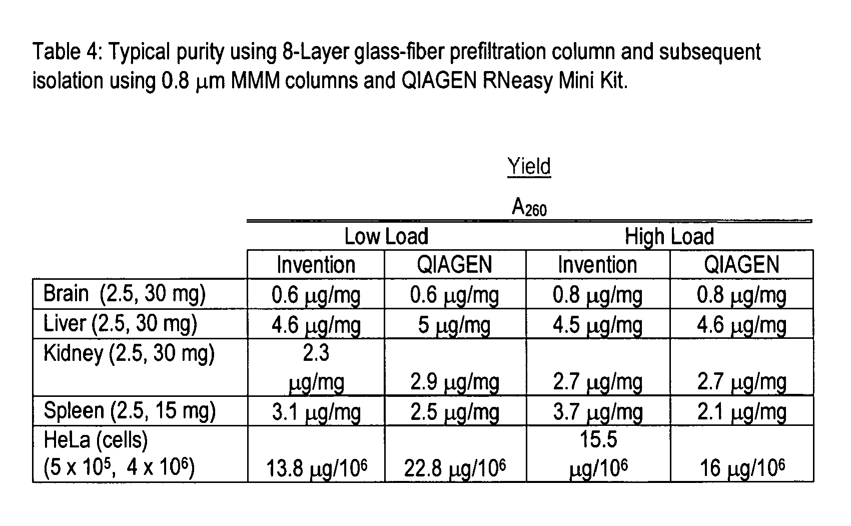

Reduction of gDNA contamination is important in many molecular biological assays, in particular, quantitative RT-PCR. RT-PCR is generally a two-step reaction or assay in which the first step is the generation of a cDNA template via a reverse transcriptase reaction. The second step is the PCR generated by a DNA polymerase such as Taq Polymerase. Genomic DNA contamination results in increased background in quantitative RT-PCR applications. This is indicated by a signal from the negative control sample. Signal from this sample results from gDNA contamination, as there is no cDNA template generated due to the omission of reverse transcriptase in the generation of template samples (thus, -RT reaction). Signal from "+RT" samples may be generated by either the cDNA or the contaminating gDNA. Thus, the "-RT" sample is important to quantitate the signal generated by the contaminating gDNA.

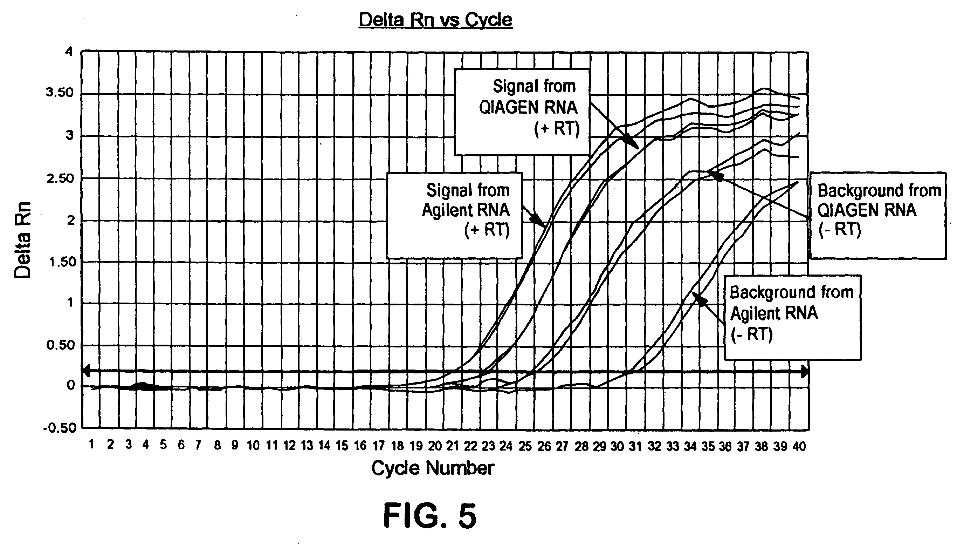

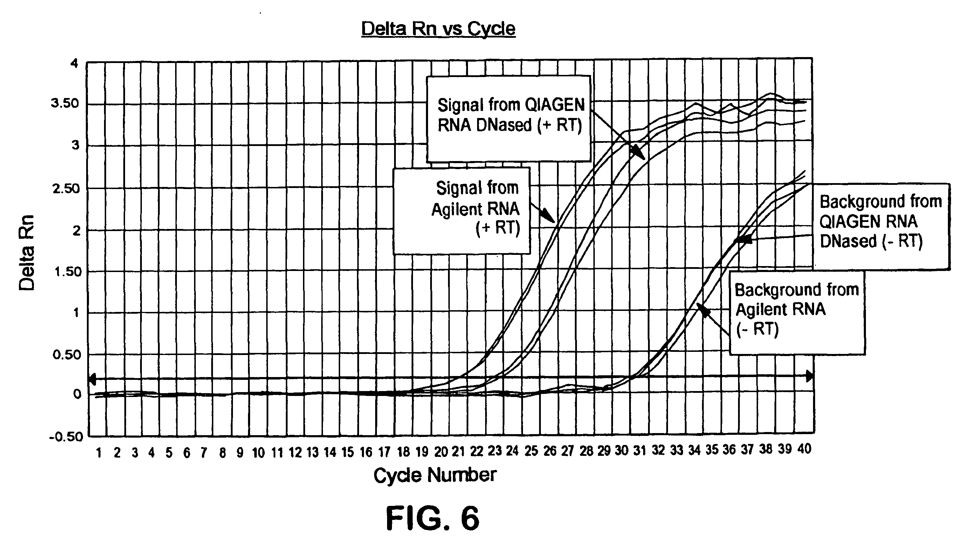

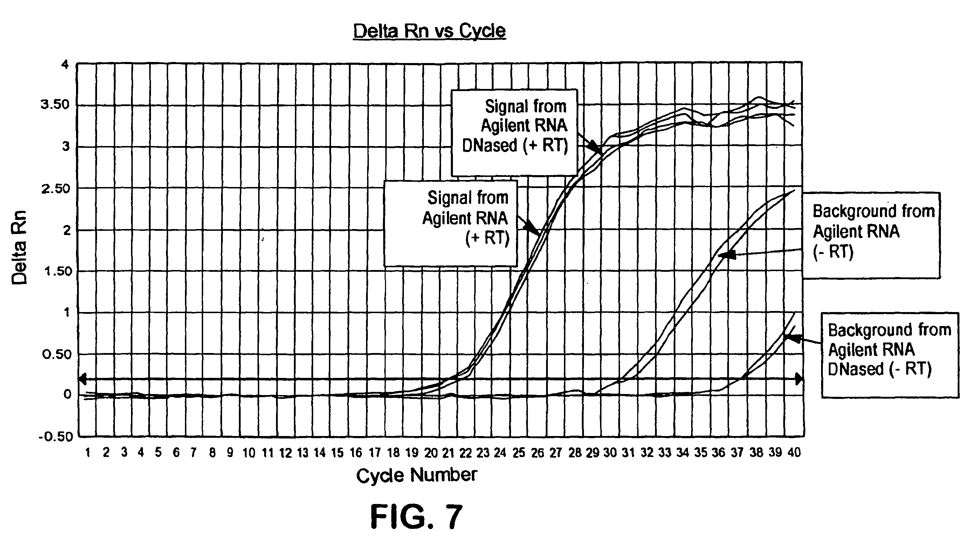

Figures 4-6 represent the output of a quantitative PCR from various spleen RNAs using the ABI 7000 and associated software. Results from the RNAs isolated with the columns and method of the present invention and the QIAGEN RNeasy Mini kit are shown in FIG. 5. The background of the reaction from isolated RNA using the columns and methods of the present invention is significantly lower than the background of the reaction from QIAGEN isolated RNA. Background of the QIAGEN samples can be reduced to the level of the present method samples using an on-column DNase digestion according to manufacturers instructions. These results are shown in FIG. 6. Background from RNA of the present method can be further reduced with on-column DNase digestion (see FIG. 7).

In one aspect of the present invention, the mechanism of RNA isolation is via precipitation. RNA, either in a purified or semi-purified (following prefiltration) form or in a complex biological sample, will precipitate in the presence of guanidine and ethanol. This precipitate can be collected via, for example, centrifugation. The RNA isolation membrane column of the present invention facilitates the collection of the RNA precipitate, washing of the collected precipitate (reduced wash volumes and centrifugation times) and re-suspension and elution of the target nucleic acid.

In some embodiments, the devices and methods may be optimized by selecting polymeric material that optimally reduce absorptive losses. For example, membranes may be selected whose pore sizes result in additional recovery of RNA. Similarly, comparison of membranes prepared from different polymeric constituents may also be used to optimize mass recovery of RNA.

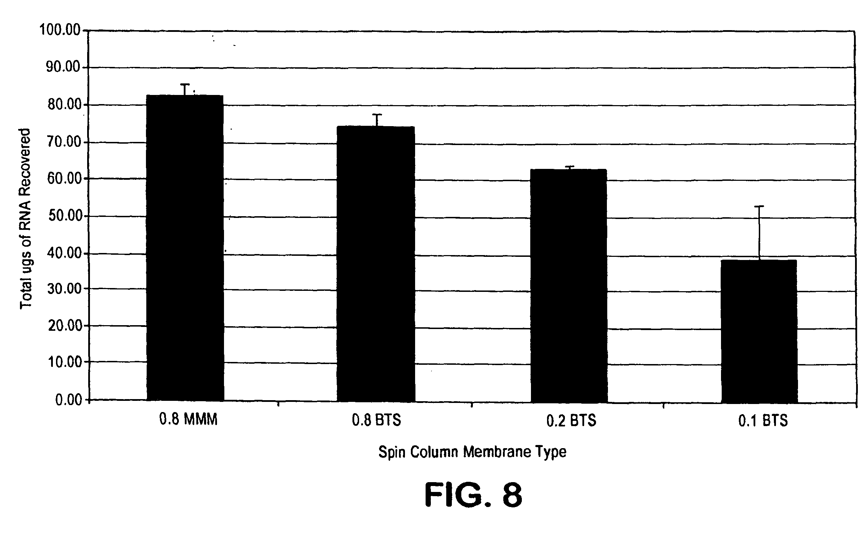

To illustrate how membrane pore size may be varied to optimize the amount of RNA recovered, a mouse spleen was homogenized and passed through a glass-fiber prefilitration column. An aliquot, 250 µL (12.5 mg), of this prefiltered homogenate was mixed with 250 µL of 70% ethanol and then loaded onto 4 different types of spin columns: (i) 0.8 µm MMM (composite of polysulfone and PVP (poly(vinyl-pyrrolidone)), (ii) 0.8 µm BTS (polysulfone with a hydroxypropyl-cellulose treatment), (iii) 0.1 µm MMM, and (iv) 0.1 µm BTS. After washing and drying said spin columns, the total RNA was eluted off each column using 50 µL of water. The recovery of RNA in the eluates was quatified by measuring absorbance at 260 nm using an Agilent model 8453 UV/Vis spectrophotometer. Figure 8 is a graphic representation of the total RNA yields derived from the O.D. 260 readings. The first two columns in FIG. 8 (data also presented in Table 10 infra) show that the MMM membrane has a distinct advantage over the BTS membrane, each of which are constructed of different polymeric materials, in the mass of RNA recovered. Columns 2, 3 and 4 show that the pore size of the membranes must be optimized for optimal performance as well.

Complementary RNA ("cRNA") also known as aRNA (amplified RNA), molecules can be synthesized and used as hybridization probes used to detect targets (usually DNA) in, for example, a microarray system. The preparation of cRNA molecules is well known to those skilled in the art. (See, for example, Agilent Technologies' "Low RNA Input Fluorescent Linear Amplification Kit," (2003) version 1.1, product # 5184-3523, the entire teaching of which is incorporated herein by reference.) During the cRNA synthesis, label is incorporated into the cRNA molecule, for example, a fluorescent label such as cyanine or can be attached later to the cRNA molecule by many different enzymatic methods.

The RNA of the present invention can be purified away from the impurities of the synthetic reaction as well as from other sources producing an isolated form of RNA. By purification it is understood that the RNA is from about 55% to about 65% pure of other non-RNA molecules. In another aspect, it is understood that the RNA is from about 65% to about 75% pure. In yet another aspect it is understood that the RNA is from about 75% to about 85% pure. In still another aspect, it is understood that the RNA is from about 85% to about 95% or greater pure. The RNA of the present invention includes mRNA, tRNA, cRNA and alike. Purity can be determined by those skilled in the art employing technologies well known to the skilled artisan such spectrophotometry, gel analysis, and alike.

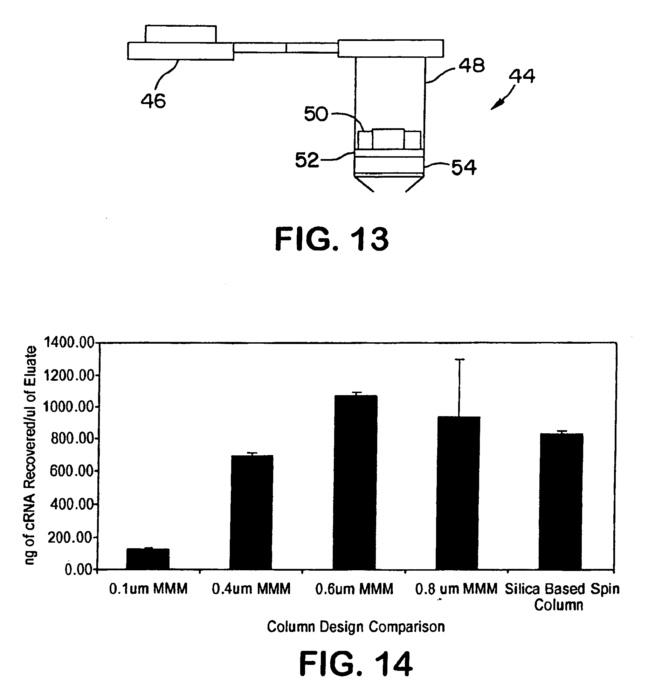

Once the cRNA molecule is synthesized, with or without a label molecule, it can be purified from contaminants using a cRNA isolation column of the present invention. The cRNA isolation column 44 of the present embodiment comprises a removable cap 46 that can be inserted onto the spin tube body 2 thus occluding only the entry orifice. See, FIG. 13. The column 44 further comprises a retainer ring 50 that assists in securing a membrane 52 into position adjacent to a frit 54. In one aspect, the membrane is an MMM membrane. In one aspect, the column comprises an asymmetric membrane made up of alloys of polysulfone and Polyvinylpyrrolidone. MMM membrane has 30-40 µm diameter pores on an upper side and 0.6 µm diameter pores on the lower side (the upper side of the membrane is in contact with the retainer ring 50, the lower side is in contact with the frit 54). The column membrane captures the cRNA thus allowing the contaminants to be washed away using appropriate buffers. Following this washing step(s), the cRNA can be eluted from the column in a purified form.

One aspect of the present method comprises adding a predetermined amount of nuclease free-water to the cRNA sample. In one aspect, around 20 µL of nuclease free-water is added to bring the total volume up to around 100 µL. To this is added approximately 350 µL of Stabilization Solution (see p 23 of this patent app.) and the mixture is mixed thoroughly. To this mixture, approximately 250 µL ethanol(~96-100% purity) is added and mixed thoroughly by, for example, pipet mixing. Transfer approximately 700 µL of the cRNA sample to a cRNA isolation column of the present invention, for example, a MMM column. The sample can now be centrifuged for about 30 seconds at around 13,000 rpm. The flow-through can be discarded. About 500 µL of Wash Buffer #3 (see p 23 of this patent app.) can be added to the column. The sample can once again be centrifuged for about 30 seconds at around 13,000 rpm. The flow-through can be discarded. Again, about 500 µL of Wash Buffer #3 (see p 23 of this patent app.) can be added to the column. The sample can once again be centrifuged for about 30 seconds at around 13,000 rpm. The flow through can be discarded. The cRNA sample can be eluted at this point. Preferably employing a new collection tube (~1.5 mL), approximately 30 µL of RNase-free water can be added to the column. After a sufficient time, for example, 1 minute, the sample can be centrifuged for about 60 seconds at approximately 13,000 rpm. This time it is important to collect and save the flow-through. One may repeat this step again by adding approximately 30 µL of RNase-free water to the column and proceed as before. The collected flow-through can be stored at this point at about -80° C. The cRNA product can be quantitated using techniques well known to those skilled in the art (see, Agilent Technologies' Amplification Kit manual).

A kit is also an embodiment of the present invention. The kit of the present invention comprises at least one prefiltration column. In one aspect, the pre-filtration column is constructed using a fiber material, such as glass or borosilicate fibers. The kit also comprises at least one RNA isolation membrane column. The membrane in this RNA isolation column can include, without limitation, BTS, PVDF, nylon, nitrocellulose, polysulfone, MMM, PVP, and composites thereof. Reagents are also part of the kit of the present embodiment. The reagents include at least one organic solvent and at least one lysis buffer (such as described herein). Other reagents can be included with in the kit. Instructions for a practitioner to practice the invention is also included.

Another kit is also an embodiment of the present invention. This kit comprises at least one cRNA isolation membrane column. The membrane in this cRNA isolation column can include, without limitation, BTS, PVDF, nylon, nitrocellulose, polysulfone, MMM, PVP, and composites thereof. Reagents are also part of the kit of the present embodiment. The reagents include at least one organic solvent and at least one stabilization buffer (such as described herein). Other reagents can be included with in the kit. Instructions for a practitioner to practice the invention is also included.

Purification of total RNA from plant tissue has traditionally been hampered by the wide range of plants and tissue types in existence. Successful RNA isolation can suffer from several problematic components, including: nucleases, polyphenolics and other oxidants; waxes, oils, resins, and latexes; and high polysaccharide content. Currently, isolation procedures include aqueous/organic solvent extraction (e.g., Triazol from Invitrogen or TRI Reagent from Sigma), or aqueous chaotropic extractions with silica-based solid phase devices for binding and purification (e.g., Rneasy from Qiagen™ or RNAqueous™ from Ambion).

The methods described herein are easy, rapid, qualitative and quantitative methods for isolating RNA virtually free of gDNA and are particularly amenable to the isolation of plant RNA. Presenting one aspect, the methods employ an aqueous buffer comprising guanidine hydrochloride in conjunction with at least one spin column. In another aspect, the method includes two spin columns; a pre-filter that removes contaminating gDNA, cell debris and other contaminants followed by an RNA filter that captures the purified RNA. Typically, RNA yields are equal to or greater than silica based aqueous extraction methods and absorbance ratios at 260/280 and 260/230 are well above 1.8, indicating that the samples are free of protein and polysaccharide contamination, respectively. DNA contamination of RNA isolated with the pre-filter is around 1000 to 10,000-fold lower than samples isolated without the pre-filter.

A typical protocol includes the following: a tissue sample is collected and processed immediately or flash frozen in liquid nitrogen. The frozen sample can be stored at -70° C. The weight of the sample is obtained then the tissue is placed in an appropriate container that comprises a lysis solution. Approximately 10 µL of lysis solution per milligram of tissue to be homogenized can be used. For desiccated or lyophilized tissue, approximately 20-40 µL per milligram of tissue can be used. The tissue can be homogenized using a conventional rotor-stator homogenizer (or any suitable homogenizer) at 15,000 rpm for 30 seconds. To reduce foaming, the probe can be moved from side to side rather than up and down. Larger volumes (*e.g.*, more than 10 mL) or fibrous tissues may require slightly longer homogenization times. The preparation can be stored at this juncture at - 70° C.

The homogenate (around 400 µL) can be centrifuged through a prefiltration column for about 3 minutes at full speed (~ 16,000 x g). This step helps ensure complete homogenization of the tissue and remove cellular contaminants including gDNA. It is important to note, that mini prefiltration columns should not be reused. For a majority of prefiltration columns, the maximum volume is about 600 µL. If processing more than 600 µL, one should employ a new prefiltration column.

An equal volume of isopropanol (or ethanol or alike) is added to the filtrate and mixed until the solution appears homogenous. For certain tissues, the resultant mixture may appear opalescent - this is not a problem. However, a very large amount of white precipitate indicates the presence of carbohydrate.

Approximately 700 µL of isopropanol/lysis mixture can be added to an isolation column, then centrifuged for about 30 seconds at 16,000 x g. The flow-through can be discarded, replace the RNA-loaded column in the collection tube. If the homogenate/alcohol mixture exceeds 700 µL, add aliquots successively onto the isolation column, then centrifuge and discard the flow-through.

Approximately 700 µL of wash solution can be added to the isolation column, then centrifuge for 30 seconds at approximately 16,000 x g. Discard the flow-through, then replace the isolation column in the same collection tube. Optionally, this step can be repeated.

The isolation column can be subjected to centrifugation for approximately 1 minute at 16,000 x g. Optionally, the tubes can be open and cover them with a paper towel to prevent airborne contaminants from entering and allow to air dry for approximately 5 minutes. It is important to completely dry the isolation column to ensure that residual alcohol is not carried over during the elution.

The isolation column can now be transferred to a new Rnase-free final collection tube. Add approximately 20-50 µL of nuclease-free water. After approximately 1 minute, centrifuge for approximately 1 minute at around 16,000 x g. Other buffers, such as 10 mM Tris pH 7 to 8, 1 mM EDTA (TE) or 10 mM Tris pH 7 to 8 may be used, however, these buffers may effect some downstream applications.

In one aspect, the extraction buffer comprises about 4 to about 6 M guanidine hydrochloride and about 10 to about 100 mM Bis-Tris having a pH around 4 to about 8. The buffer also comprises about 1 to about 50 mM EDTA and about 0.1% to about 2% β-mercaptoethanol.

In a particular aspect, the extraction solution comprises approximately 5.5 M guanidine hydrochloride, about 50 mM Bis-Tris pH around 6.6, about 10 mM EDTA, and about 1% β-mercatoethanol. In another aspect, the wash solution comprises about 70% ethanol, 10 mM Tris pH ~ 8.0, and about 1 mM EDTA.

The extraction buffer described herein has been optimized for plant leaf material and several other plant tissue types. The present method also employs a prefilter (e.g., a glass fiber-based prefilter) that removes gDNA and other cellular debris. The prefiltration step replaces the more typical step of centrifugation that pellets cell debris to the tube bottom where subsequent removal of the supernatant adds an extra step to the procedure and can lead to contamination with loose pellet debris. The absence of gDNA can be very important for downstream applications such as QPCR, RT-PCR and microarray analysis. Electrophoretic and QPCR analysis demonstrate that RNA isolated using the present method generally results in 100 to 10,000 times less DNA contamination than typical organic solvent or silica based methods.

The instant method employs the same prefiltration and RNA capture columns described herein.

The instant method employs the same pre-filtration and RNA capture columns described herein.

Purification of total RNA from plant tissue has traditionally been hampered by the wide-range of plants and tissue types available. Aqueous phase isolation procedures are a popular type of commercial isolation kit due to their ease of use and rapid protocol. However, these kits are typically limited in the range of plant tissue types susceptible to isolation procedures incorporated in these kits. Particularly troublesome samples are those high in wax, oil or polysaccharides (especially storage tissue which accumulates oils and polysaccharides). Currently, some commercially available aqueous spin column kits completely fail with resinous material, such as pine. The only commercial alternative is the organic extraction -based RNA isolation kits, which contain dangerous chemicals, are tedious, and result in products heavily contaminated with gDNA.

In one embodiment, the invention provides methods for isolating nucleic acids using polyethylene glycol (PEG). In one aspect of the present embodiment, the nucleic acid that is isolated is RNA. Methods of the present embodiment include PEG in conjunction with one or more spin columns described herein used for the isolation of nucleic acids from tissue samples high in waxes, oils, latexes, and resins. In one aspect, the tissue sample originates from a plant. In one particular aspect of the instant embodiment, a prefiltration step that removes much of the contaminating cell debris and gDNA is included.

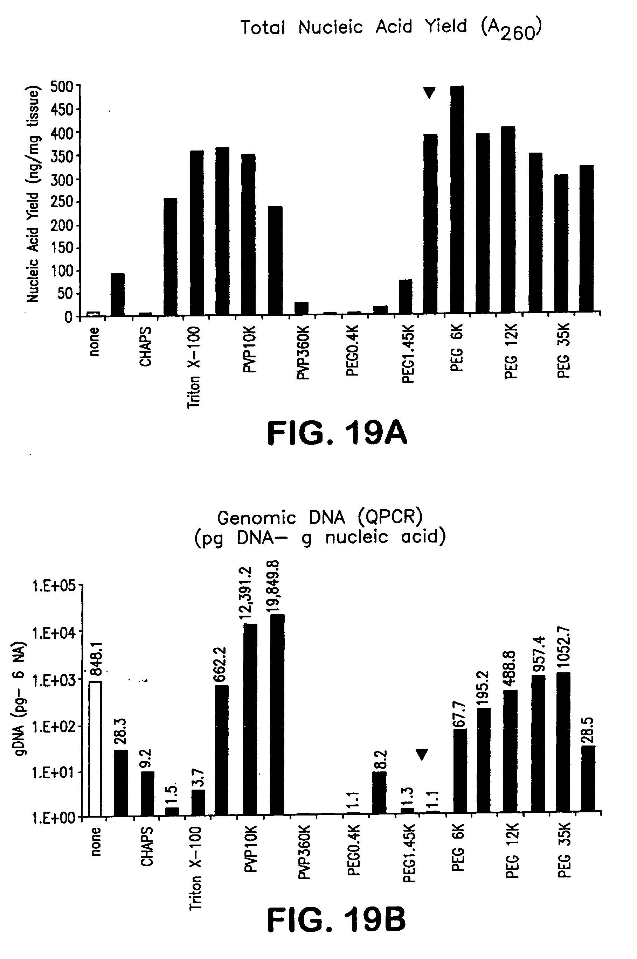

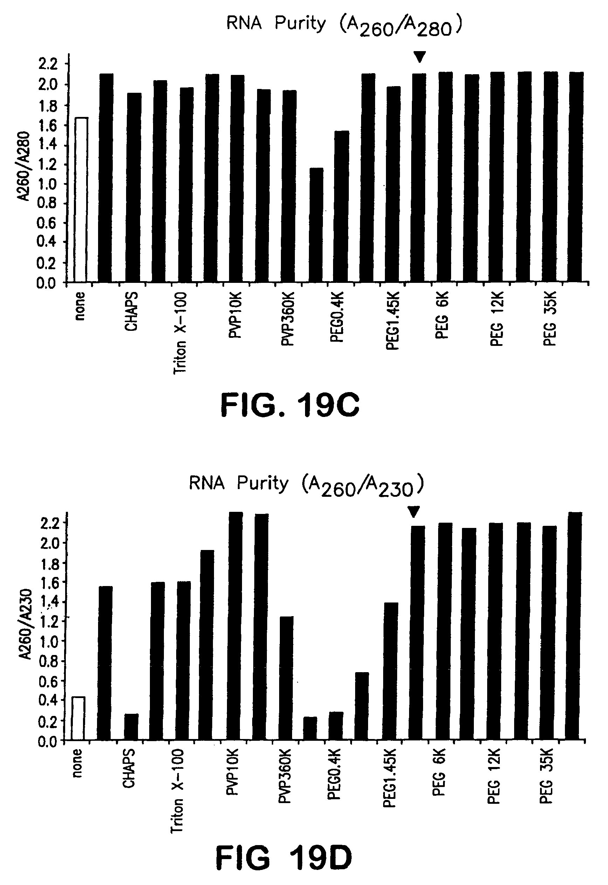

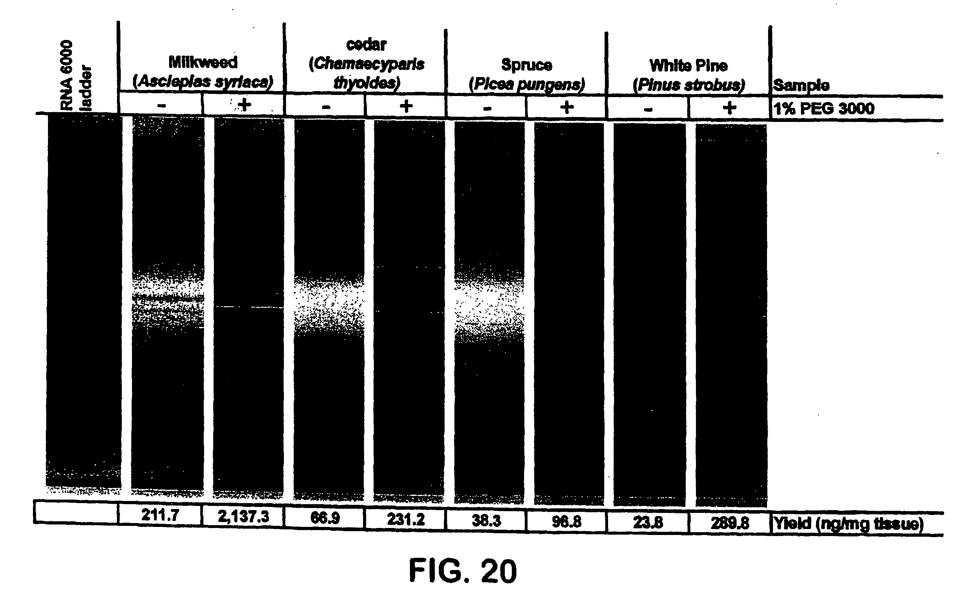

Polyethylene glycol ("PEG") increases RNA yield from, for example, pine needles, while maintaining purity and low gDNA contamination. Data from Example 16, specifically figures 19 and 20, illustrate RNA yields, gDNA contamination and RNA purity as measured by the absorbance ratios A260/A280 and A260/A230 from pine needles isolated using various detergents and PEGs. RNA was isolated from approximately 40 mg of White Pine needles.

Detergents do not appear to work well because they either reduce yield or do not significantly improve the A260/A230 ratio, which is indicative of carbohydrate contamination (see Example 16). Low molecular weight polyvinylpyrrolidones increases yield and purity but gDNA contamination tends to be high. However, the use of low molecular weight polyvinylpyrrolidinones may be acceptable when gDNA is isolated or when it is unnecessary to remove gDNA from a sample. High molecular weight polyvinylpyrrolidone and polyvinylpolypyrrolidone do not enhance RNA isolation. PEG enhances RNA yields and purity while maintaining low gDNA contamination. RNA quantity and quality increases with increasing molecular weight (~3000 to 35,000 daltons).

In one aspect, the method comprises a method of preparing an RNA sample from a tissue having resin. This method includes forming an extraction preparation using an extraction buffer. In one aspect, this extraction buffer comprises polyethylene glycol. In a particular aspect, the extraction buffer includes between 0.25 to about 5% polyethylene glycol. In another aspect, the extraction buffer comprises polyethylene glycol and a chaotropic agent. For example, in one aspect, the Extraction Buffer can include about 0.1 to about 2% β-mercaptoethanol, about 4 to about 6 M guanidine hydrochloride, about 10 to about 100 mM bis-tris HCl (pH ~ 4-8), and about 1 to about 50 mM EDTA. The extraction preparation can then be introduced to a prefiltration column of the present invention forming an RNA solution. This RNA solution can next be introduced to an isolation column of the present invention. The adsorbed RNA can then be eluted from the isolation column using an appropriate elution solution

In particular, PEG3000 produces significant RNA yields and purity with very low gDNA. The invention is also directed to a kit used for the isolation of nucleic acid from a tissue sample. In one aspect the tissue sample originates from a plant. In another aspect, the tissue sample originates from a plant. The present kit includes reagents and columns used to isolate nucleic acid from a tissue sample. The reagents of the present comprise polyethylene glycol. In addition to PEG, in certain aspects, the reagents of the present kit comprise Extraction Buffer and Plant Wash solution. The kit of the present embodiment further comprises one or more spin columns of the instant invention and one or more prefiltration columns of the current invention.

### EXAMPLES

The following examples are provided to illustrate uses of devices and methods according to certain aspects of the invention and not intended to not limit the scope of the invention.

### Example 1: Component Preparation

### (a) Silicon Carbide Whisker column Preparation

Silicon Carbide whiskers were obtained from Surmet (Buffalo,NY) and slurried in an aqueous solution. A spin-column device (Orochem, Westmont, IL) was placed on a vacuum manifold and a polyethylene frit of about 7 µm in pore size (Porex Corp., Fairburn, GA) was placed in the spin column device. A slurry of SiCw was placed in the spin column and vacuum was applied. The column was allowed to dry slightly with vacuum. A plastic retainer ring was placed on the bed of silicon carbide whiskers to secure the spin column.

### Fiber filter Column Preparation

In this particular example, Whatman GF/F Glass Fiber Filters (cat no. 1825-915) were purchased from Fisher Scientific (Atlanta, GA). Multiple layers (of the large sheets or disks supplied) were punched with a 9/32" hand punch (McMaster-Carr, Chicago, IL) to form the pre-filters of the present invention, and placed into a spin column (Orochem, Westmont, IL) fitted with a 90 µm polyethylene frit (Porex Corp., Fairburn, GA) on which the fibers rest. The filter materials were secured in the column with a firmly-placed retainer ring on top of the filter materials to prevent excessive swelling of the fibers (Orochem, Westmont, IL). For example, see FIG. 2.

### Reagent Preparation

The following solutions were prepared or obtained from commercial sources for use in the procedures performed in the remaining example below. All reagents prepared were prepared in nuclease-free H₂O and stored at room temperature except for the Lysis Solution, DNase I and Proteinase K. The Lysis Solution with β-Mercaptoethanol was stored at 4° C. DNase I and Proteinase K were stored at -20° C.

### Lysis Buffer/Solution Stock:

4 M Guanidine Thiocyanate (Sigma, St. Louis, MO)
25 mM Tris, pH 7 (Ambion, Austin, TX)
To make a working solution, β-Mercaptoethanol was added to a concentration of 143 mM.

### Stabilization Solution:

0.2 to 5 M Guanidine Thiocyanate, 10 to 150 mM Tris, pH 6 to 8.

### Wash Buffer #1:

From about 0.2 to about 2 M, *e.g*., 1 M Guanidine Thiocyanate (Sigma, St. Louis, MO) 25 mM Tris, pH from about 6 to about 9, *e.g.*, 7 (Ambion, Austin, TX) from about 5 to about 25% ethanol, *e.g.,* 10% ethanol (Sigma, St. Louis, MO).

### Wash Buffer #2:

Twenty-five mM Tris, pH from about 6 to about 9, *e.g.,* 7 (Ambion, Austin, TX) from about 40 to about 90% ethanol, *e.g.,* 70% ethanol (Sigma, St. Louis, MO)

### Wash Buffer #3:

Five to 250 mM Tris, pH from about 6 to about 9, from about 40 to about 90% ethanol.

### QIAGEN® reagents:

RLT, RW1, RPE buffers were prepared according to manufacturer's instructions (RNeasy Mini Kit, part no. 74104, Valencia, CA).

### DNase I:

RNase-free DNase I was obtained from Ambion, Austin TX.Proteinase K was obtained from Fermentas, Hanover, MD.

### DNase digestion buffer 10X:

1M Tris pH 8 (Ambion, Austin, TX)
100 mM MgSO4 (Sigma, St. Louis, MO)
100 mM CaC12 (Sigma, St. Louis, MO)
1 mg/mL Bovine Serum Albumin (Sigma, St. Louis, MO)

### Elution Buffer:

Three examples of such elution solutions are 10 mM EDTA and 10 mM sodium citrate, pH ranging from 6 to 9 as well as free-nuclease water.

### Example 2: RNA isolation from mouse tissues

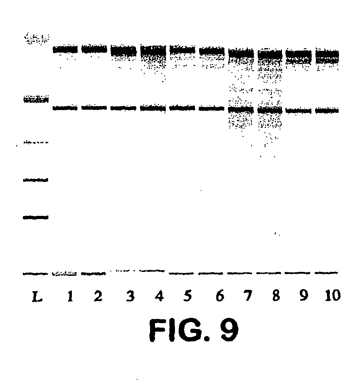

The experiments below describe side by side RNA Isolations from a variety of mouse tissues and cells, the RNA was isolated using a SiCw column (FIG. 1). RNA was assayed with the RNA 6000 Nano Assay (Agilent Technologies, Palo Alto, CA, part no. 5065-4476) on the Bioanalyzer 2100 (Agilent Technologies, Palo Alto, CA, part no. G2938B) as per Manufacturer's instructions. Figure 9 and Table 5 show composite results from multiple assays - *i.e.,* for FIG. 9, not all the assays shown were run on the same chip. The legend for interpreting FIG. 9 is L: Ladder, Ambion RNA 6000 Ladder (Part Number 7152), lanes 1-2: Brain RNA isolated with SiCw column, lanes 3-4: Liver RNA isolated with SiCw column, lanes 5-6: Kidney RNA isolated with SiCw column, lanes 7-8: Pancreas RNA isolated with SiCw column, and lanes 9-10: Spleen RNA isolated with SiCw column. Table 5 is a summary of the resulting RNA yields.

Mouse organs that were quick frozen in liquid nitrogen immediately after harvest were obtained from Pel-Freez Biologicals (Rogers, AR). Alternatively, mouse organs can be used immediately after harvest, or preserved in a solution of RNALater (Ambion, Austin, TX).

Samples were weighed and power-homogenized in excess Lysis Solution (Applicants' method) having a pH in the range of about 4 to about 8 (typically 20-fold excess) at 15,000 rpm for 30 seconds using an OMNI TH tissue homogenizer (Omni, Inc, Warrenton, VA).

Cell lines were obtained from American Type Tissue Collection (ATCC, Manassas, VA 20108) and grown according to instructions provided. (See Table 5.) Cells were trypsinized to detach from the culture vessel, resuspended and counted with a hemocytometer. The suspension was then centrifuged at 1,000 x g for 10 minutes. The resulting pellet was resuspended in Lysis solution for a final concentration of 8.3 x 10⁶ cells/mL and vigorously vortex mixed for 1 min. Alternatively cells may be lysed in the cell culture vessel.

Tissue or cell homogenate (typically 300-600 µL) was added to a glass-fiber pre-filter in a spin column and centrifuged for 3 min. at 16,000 x g in an Eppendorf 5415D microcentrifuge (Brinkman, Westbury, NY).

An equal volume of 70% ethanol was added to each of the tissue homogenates and mixed. Spin columns containing 15 mg of silicon carbide whiskers (SiCw) were placed in capless 2 mL collection tubes, and the ethanol-containing homogenates were added to the spin columns. The spin columns were then spun for at least 10 sec. at 16,000 x g. The flow-through was decanted from the collection tubes and the spin columns placed back in the collection tubes.

The spin columns were then washed with 500 µL Wash Buffer #1 and centrifuged for at least 10 sec. at 16,000 x g. Following an extended centrifugation, the spin column could be subjected to DNase digestion. When DNase digestion was not performed, each spin column was then centrifuged twice for at least 10 sec. at 16,000 x g with the addition of 500 and 250 µL of Wash Buffer #2. The spin columns were then centrifuged for 2 min. at 16,000 x g to remove the final traces of Wash Buffer #2. The spin columns were removed from the 2 mL collection tubes and placed into 1.5 mL nuclease-free microfuge tubes.

RNA was eluted twice with 50 µL nuclease-free water, centrifuging 15 sec. and 2 min. respectively. RNA can then be stored at -20° C or -70° C.

Absorbance at 260 nm and 280 nm was measured on an Agilent Technologies 8453 UV/VIS spectrophotometer to confirm the presence of eluted RNA.

RNA obtained in the experiments was assayed with the RNA 6000 Nano Assay (Agilent Technologies, Palo Alto, CA) per manufacturer's instructions, as show in the software generated images in FIG. 9.
As can be seen in Table 5 and FIG. 9, RNA purified using the methods and devices of the present invention is of high yield, purity and integrity.

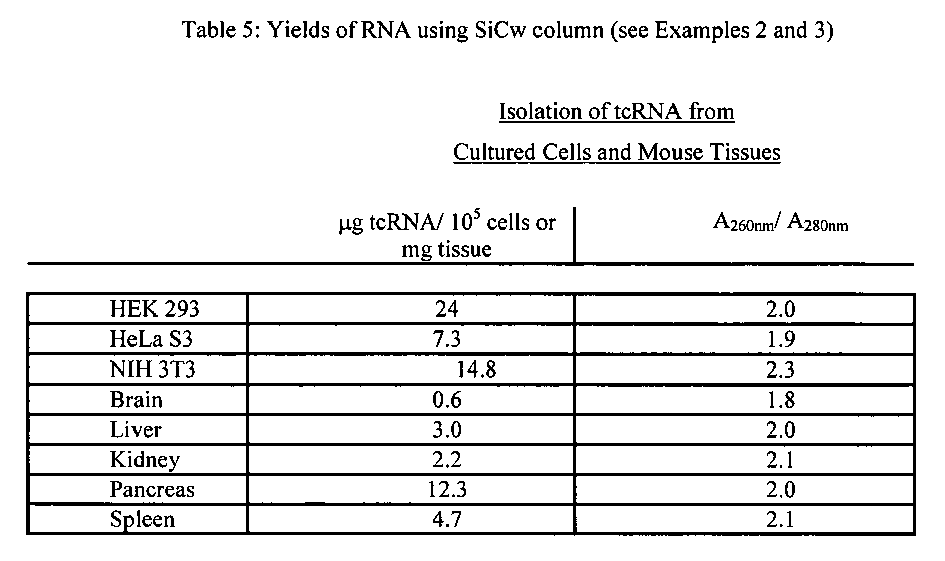

Example 3: The experiment below describes side by side RNA Isolations using a variety of glass fiber type filter materials.

For pre-filtration devices and the attendant methods, a variety of 16-layer glass fiber filter types were constructed. Whatman Types GF/F (cat no. 1825-915) and GF/D (part number 1823-150) were obtained from Fisher Scientific (Atlanta, GA). Pall Life Sciences Types A/B (part number 66211) and A/D (part number 66227) were obtained from VWR (Pittsburg, PA). For those samples on which DNase digestion was performed, the on-column DNase digestion methods outlined below were used.

Subsequent purification of the samples was performed by either a silica-based method of QIAGEN® per manufacturer's instructions, or by silicon carbide whisker methods and devices of the instant invention. In addition, on-column DNase digestion methods were used. The resulting RNAs were assayed with the Agilent Technologies' RNA 6000 Nano assay (part no. 5065-4476) on the Bioanalyzer 2100 (part no. G2938B, Agilent Technologies, Palo Alto, CA) as per manufacturer's instructions. Figure 9 is the software-generated results of the electrophoresis assay results.

Mouse spleens that were quick frozen in liquid nitrogen immediately after harvest were obtained from Pel-Freez Biologicals (Rogers, AR). Spleen tissue was selected for the examples shown herein because spleen tissue is one of the most difficult tissues from which to isolate RNA due to the large amounts of gDNA present in the spleen. Spleen RNA was isolated, following pre-filtration, using silicon carbide whiskers (SiCw) and/or silica-based (QIAGEN) isolation methods.

Samples were weighed and power-homogenized in excess Lysis Buffer (presented above) having a pH in the range of about 4 to about 8, or RLT (typically 20-fold excess) from QIAGEN and subjected to 15,000 rpm for 30 seconds using an OMNI TH tissue homogenizer (Omni, Inc, Warrenton, VA).

Tissue homogenate (typically about 300-600 µL) was pre-treated for use in a QIAGEN column by centrifuging for 3 min. at 16,000 x g, or pre-treated for use in a SiCw column by adding it to a glass fiber pre-filter of the present invention and then centrifuged for 3 min. at 16,000 x g in an Eppendorf 5415D microcentrifuge (Brinkman, Westbury, NY).

An equal volume of 70% ethanol was added to each of the tissue homogenates and mixed. The ethanol-containing homogenates were then added to either a mini spin-column from QIAGEN's RNeasy Mini Kit (Valencia, CA, part no. 74104) or to a SiCw spin-column described herein.

The spin columns were then centrifuged for at least 10 sec. at 16,000 x g. The effluent from each was collected in and decanted from 2 mL collection tubes and the spin columns were placed back in the collection tubes.

The spin columns were then washed with 700 µL RW 1 (QIAGEN RNeasy column) or 700 µL Wash Buffer #1 and centrifuged for at least 10 sec. at 16,000 x g. For those samples using the SiCw spin column, the SiCw spin column contents were then either subjected to DNase digestion as described below or washed with Wash Buffer #2 as described below.

For those columns not subjected to DNase digestion, the columns were centrifuged twice as described above (for at least 10 sec. at 16,000 x g) with the addition of 500 µL (1^{st} time) and 250 µL (2^{nd} time) of RPE (a QIAGEN RNeasy column buffer) or Wash Buffer #2. The second centrifugation was extended to 2 min. at 16,000 x g to remove the final traces of RPE or Wash Buffer #2. Each of the columns were then removed from its 2 mL collection tube and placed into a 1.5 mL nuclease free microfuge tube. RNA was eluted twice using 50 µL nuclease-free H₂O. RNA was then stored at or - 70°C.

As noted above, for example, before the addition of Wash Buffer #2, samples can be optionally subjected to DNase treatment. For those columns that were subjected to DNase treatment, DNase I was diluted to a concentration of 0.5 µg/µL in 100 µL final volume in 1X DNase buffer and applied to the SiCw column. (Note that the methods of the present invention described herein use low salt concentrations in the DNase buffer, for example, as low as 100 mM, whereas most conventional methods require a high salt concentration - for example, up to as much as 1M NaCl is used in some commercial DNase buffers for on-column digestion.) The methods of the instant invention do not use salt to increase ionic strength or retain binding because DNase is very sensitive to high ionic concentrations (note the reagents listed in Example 1, supra), for example, only 100 mM CaCl₂ was used in the present example. The column was then incubated for 15 min. at 25°C. The digestion was terminated by the addition of 500 µL of Wash Buffer #1 and centrifuging for at least 10 sec. at 16,000 x g. Washing with Wash Buffer #2 was performed and then the elution was performed as described above after the final traces of Wash buffer #2 were removed. Samples on which the QIAGEN method was used were not subjected to DNase digestion.

Genomic DNA contamination was quantified using a 5' nuclease assay, or "real-time" PCR assay, run on the Applied Biosystems Prism 7000 Sequence Detection System (Applied Biosystems, Foster City, CA). This type of assay monitors the amount of PCR product that accumulates with every PCR cycle. This is a highly sensitive and reproducible assay for the detection of PCR product.

Isolated tcRNA (~20 ng) from mouse mouse spleen was added to a reaction mixture containing primers and probe specific for mouse (Genbank Accession NM-008084) glyceraldehyde-3-phosphate dehydrogenase (GAPDH). All samples were run in a reaction mixture consisting of both primers at 500 nM, fluorescent probe at 200 nM, and 1X Taqman Universal Master Mix (part #43044437) in conditions well known to those skilled in the art. Serial dilutions of mouse genomic DNA (Promega, Madison WI) were used for the generation of a standard curve. All samples, standards and no-template controls were run in duplicate.

The mouse GAPDH assay amplified a 78 base-pair fragment within an exon. The GAPDH assay primers and probe were designed using the Primer Express software package (Applied Biosystems, Foster City, CA,part no. 4329442). The primers were desalted and the probe (5' labeled with 6-FAM and 3' labeled with BHQ-1) was purified by anion exchange followed by reverse phase HPLC (Biosearch Technologies, Novato, CA).

The assay cycling parameters for both assays were the default conditions set by the manufacturer, *i.e.*, 50°C for 2 min., 95°C for 10 min., then 40 cycles of 95°C for 15 sec. to 60°C for 1 min. Quantification of gDNA in the isolated tcRNA was calculated from the mouse gDNA standard curve.

Table 6 shows the results of the quantitative PCR assay demonstrating the reduction of gDNA via pre-filtration and/or on-column DNase digestion, in various combinations of centrifugation only, pre-filtration, DNase digestion, QIAGEN methods, and the SiCw methods of the present invention. Table 7 lists the RNA yields and gDNA contamination of the spleen tcRNA experiments shown in FIG. 9. Yields are not shown for samples with high gDNA contamination. Levels of gDNA were determined by the real time PCR assay described in above example.

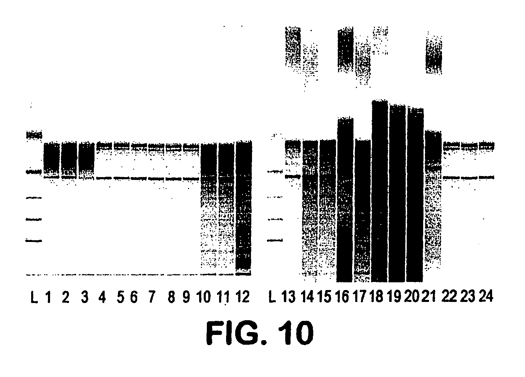

Figure 10 illustrates the high levels of gDNA contamination detected in the Agilent RNA 6000 Nano assay, as seen in lanes 1-3, 10-12, 16-18, and 19-21 vs. the low levels for example in lanes 4-6. The legend for FIG 10 is L: Ladder, Ambion RNA 6000 Ladder (part no. 7152), lanes 1-3: Spleen RNA isolated with SiCw column from cleared (centrifuged) homogenate, 4-6: Spleen RNA isolated with SiCw column from GF/F pre-filtered homogenate, 7-9: Spleen RNA isolated with SiCw column from GF/F pre-filtered homogenate and subjected to on-column DNase digestion, 10-12: Spleen RNA isolated with SiCw column from GF/D pre-filtered homogenate, 13-15: Spleen RNA isolated with SiCw column from A/B pre-filtered homogenate, 16-18: Spleen RNA isolated with SiCw column from A/D pre-filtered homogenate, 19-21: Spleen RNA isolated with RNeasy mini column from cleared (centrifuged) homogenate, and 22-24: Spleen RNA isolated with RNeasy column from GF/F pre-filtered.

This assay clearly demonstrates that only particular types of fiber and filters effectively remove gross gDNA contamination. For example, using the Whatman GF/F filter material proved most effective, as shown in lanes 4, 5, and 6 where there is very little gDNA contamination in the final eluted samples and no DNase digestion was performed. Similarly, in lanes 22, 23, and 24 in which the QIAGEN procedures were augmented by the pre-filtration methods of the present invention with glass fiber filters, there is significantly less gDNA contamination than is present in all of the other samples/lanes in which QIAGEN procedures were used.

In contrast, lanes 10-12 and 16-18 have considerably more gDNA contamination. Lanes 10-12 used a filter material having a particle-retention of about 3 µm, and lanes 16-18 used a filter material having a particle-retention of about 1 µm, while the filter material used for lanes 4, 5, and 6 and the filter material used for lanes 22, 23, and 24 had a particle retention of about 0.7 µm. Therefore, filter type, composition and performance must be optimized.

Referring to Table 6 and FIG. 10, it can be observed from lanes 1-3, 10-12, 16-18 and 19-21 without DNase digestion, there is a great deal of gDNA contamination that the RNA quantification was essentially meaningless. However, it can be observed from the results of lanes 4-6 and 22-24 that the pre-filtration methods and devices of the present invention results in essentially negligible gDNA contamination. In fact, the pre-filtration resulted in approximately the same RNA yields as those obtained with DNase treatment, without having to perform DNase treatment and without having a significant amount of gDNA present after pre-filtration than remains after DNase treatment. Compare the RNA yields and gDNA amounts of lanes 4-6 which use the pre-filtration methods and devices of the current invention to those of lanes 7-9 which use the pre-filtration methods in combination with traditional DNase treatment. While somewhat more gDNA is removed using the combination of pre-filtration and DNase digestion, the methods and devices of the present invention alone removes substantially all of the gDNA and allows a practitioner to avoid DNase treatment if desired for a particular application.

DNase digestion may be performed using methods known in the art . However, use of DNase digestion is always dependent on the end use application. For example, DNase digestion is probably unnecessary for Northern hybridizations. Therefore, whether or not DNase digestion is used or needed depends on the end application for which the RNA is being isolated.

Thus it has been shown that the methods and devices of the present invention effectively remove gDNA from a sample from which RNA is being isolated, can avoid the necessity of DNase digestion (yet are compatible with DNase digestion if necessary), and function with commercial RNA isolation kits (for example, QIAGEN's RNeasy kit), especially silica-based kits, to enhance their effectiveness.

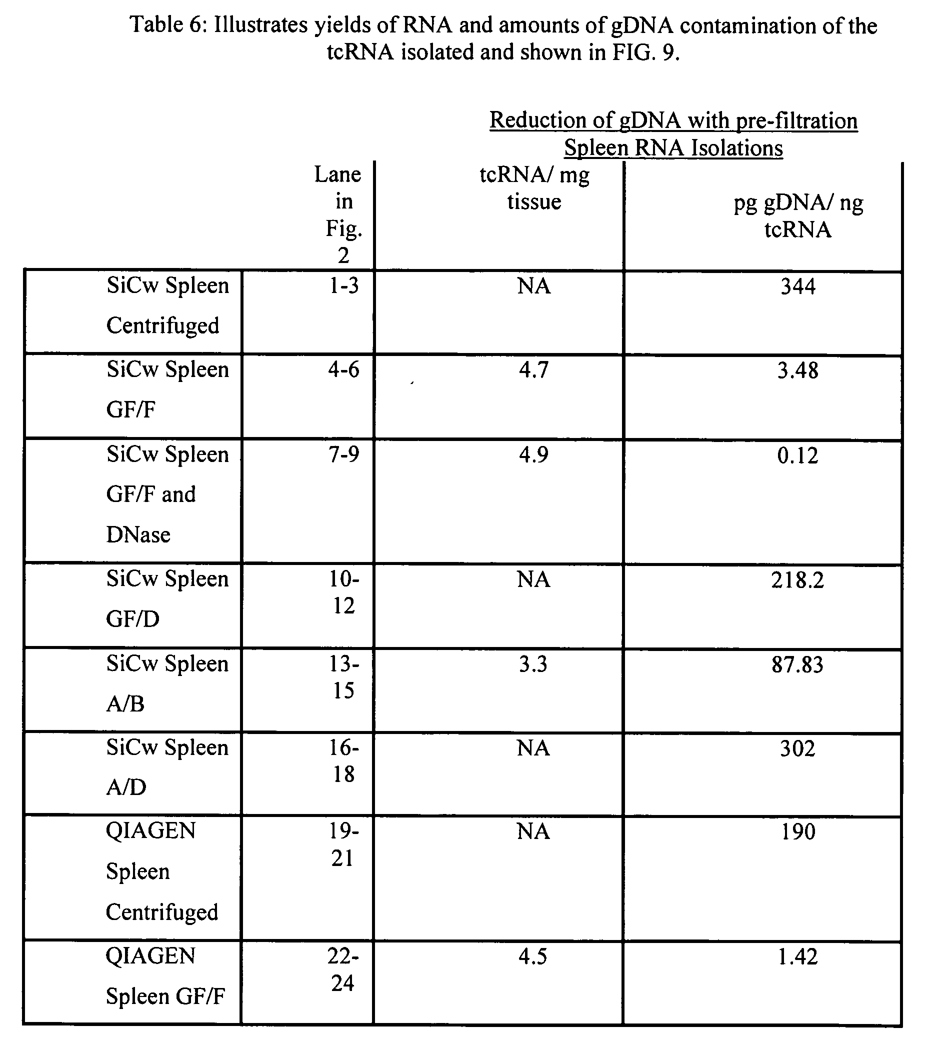

### Example 4: Mouse liver homogenate

Mouse liver homogenates were prepared as described herein and used for RNA isolations. The addition of 70% ethanol to the filtered homogenate, as described in Example 2, was substituted with equal volumes of RNase-free water, 70% isopropanol or methanol. The mixture was added to a SiCw spin-column, and RNA isolation continued as described herein.

Absorbance at 260 nm and 280nm was measured on an Agilent Technologies 8453 UVNIS spectrophotometer to confirm the presence of eluted RNA.

The results of these experiments are shown in Table 7.

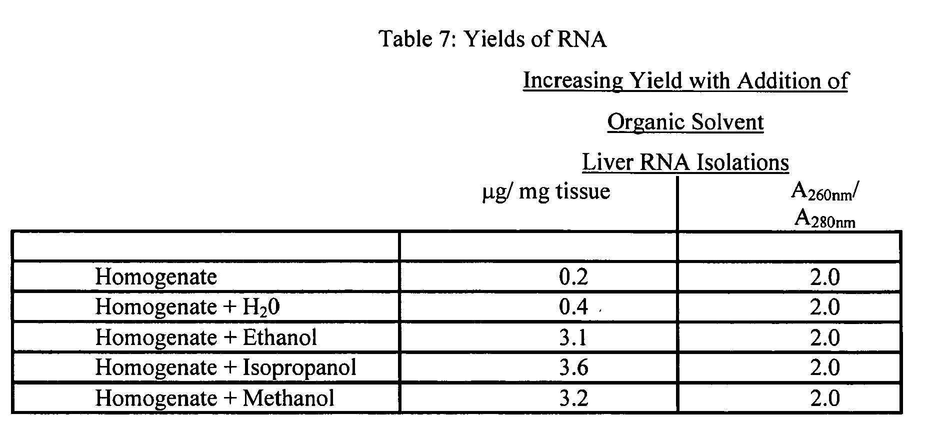

### Example 5: RNA isolation from plant tissues

Arabidopsis leaves were weighed and power-homogenized in excess Lysis Buffer at 15,000 rpm for 30 seconds using an OMNI TH tissue homogenizer (Omni, Inc, Warrenton, VA). Leaf tissue can also be frozen after harvest and homogenized as above.

Tissue homogenate (typically about 300-600 µL) was pre-treated by centrifuging for 2 min. at 16,000 x g, or by adding it to a glass fiber pre-filter of the present invention and then centrifuging for 2 min. at 16,000 x g in an Eppendorf 5415D microcentrifuge (Brinkman, Westbury, NY).

An equal volume of 70% ethanol (an example of a binding enhancer) was added to each of the tissue homogenates and mixed. The spin columns were then centrifuged for at least 10 sec. at 16,000 x g. The effluent from each was collected in and decanted from each 2 mL collection tube and the spin columns placed back in the collection tubes.

The spin columns were then washed using 500 µL of Wash Buffer #1 and centrifuged for at least 10 sec. at 16,000 x g. Each one of the columns was then centrifuged twice as described above for at least 10 sec. at 16,000 x g with the addition of 500 µL (1^{st} time) and 250 µL (2^{nd} time) of Wash Buffer #2. The second centrifugation was extended to 2 min at 16,000 x g to remove the final traces of Wash Buffer #2. Each of the columns was then removed from its 2 mL collection tube and placed into a 1.5 mL microfuge tube. RNA was eluted twice using 50 µl nuclease-free H₂O. RNA was then stored at -20°C or -70°C.

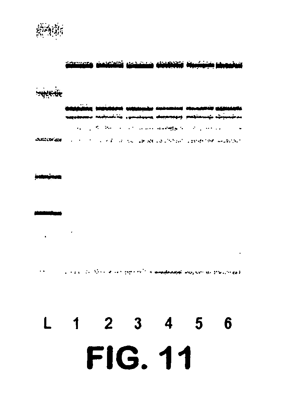

The results of the plant tissue RNA isolation are shown in FIG. 11. Resulting RNAs were assayed with the Agilent Technologies' RNA 6000 Nano assay (part no. 5065-4476) on the Bioanalyzer 2100 (part no. G2938B, Agilent Technologies, Palo Alto, CA) as per manufacturer's instructions and FIG. 5 is the computer-generated printout of the electrophoresis assay results. The legend for FIG. 5 is L: Ladder, Ambion RNA 6000 Ladder (Part Number 7152), lanes 1-3: Arabidposis RNA isolated with SiCw column from GF/F pre-filtered homogenate, and 4-6: Arabidopsis RNA isolated with SiCw column from cleared (centrifuged) homogenate.

As with the animal tissues shown in FIG. 9 and Table 7, it can be seen from the results shown in lanes 1-3 that the pre-filtration methods and devices of the present invention remove most of the gDNA from plant tissue samples. In fact, the level of gDNA present after pre-filtration was not detectable by the sensitive assays employed.

Genomic DNA contamination was quantified with a 5' nuclease assay, or "real-time" PCR assay, run on the Applied Biosystems Prism 7000 Sequence Detection System (Applied Biosystems, Foster City, CA). This type of assay monitors the amount of PCR product that accumulates with every PCR cycle. This is a highly sensitive and reproducible assay for the detection of PCR product.

Isolated tcRNA (200 ng) from arabidposis leaf was added to a reaction mixture containing primers and probe specific for the 18S ribosomal RNA. As per manufacturer's guidelines, all samples were run in a reaction mixture consisting of both primers at 500nM, fluorescent probe at 200 nM, and 1X Taqman Universal Master Mix (part no. 43044437). Serial dilutions of arabidposis DNA purified from leaves with the Promega kit were used for the generation of a standard curve. All samples, standards and no-template controls were run in duplicate.

The assay amplifies a 187 base-pair fragment within an exon. The primers and probe were designed using the Primer Express software package (Applied Biosystems, Foster City, CA, part no. 4329442). The primers were desalted and the probe (5' labeled with 6-FAM and 3' labeled with BHQ-1) was purified by anion exchange followed by reverse phase HPLC (Biosearch Technologies, Novato, CA). The assay cycling parameters for both assays are the default conditions set by the manufacturer, *i.e.,* 50° C for 2 min., 95° C for 10 min., then 40 cycles of 95° C for 15 sec. to 60° C for 1 min.

Quantitation of gDNA in the isolated tcRNA was calculated from the Arabidopsis gDNA standard curve and those results are shown in Table 8. The results shown in FIG. 11 and Table 8 demonstrate the versatility of the glass-fiber pre-filtration methods and devices of the present invention to include use with plant tissues.

Table 8: Illustrates yields of RNA and the levels of gDNA contamination of the RNA isolated and shown in FIG. 11.

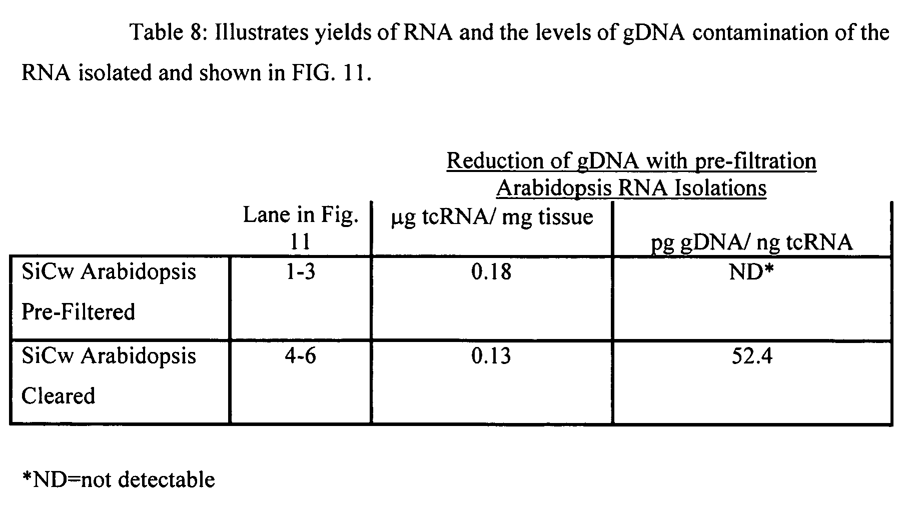

### Example 6: Additional tissue isolation

In addition to the isolation and purification procedures described above, RNA isolation from tissues high in connective tissue and contractive proteins such as skin, heart and muscle can be facilitated with Proteinase K treatment. To begin, such tissues are homogenized, as described above, and an equal volume of water can be added to the sample. Proteinase K can then be added to a final concentration of 1 unit/100 µL, mixed and incubated at 55° C for 10 minutes. The homogenate can then be centrifuged through the pre-filter column of the present invention (with/without further processing), with or without DNase treatment as described above.

Also, preparation of RNA from larger numbers of samples can be facilitated using a vacuum manifold designed for use with solid phase extraction (SPE) columns and vacuum pumps. Samples are homogenized, clarified and mixed with a low molecular weight alcohol such as ethanol, as above. If using the SiCw column of the present invention, the SiCw spin column is then placed on a vacuum manifold with the stopcock in the shut position. The ethanol-containing homogenate can then be added to the column and the stopcock opened to let the homogenate through. The stopcock is then shut, 500 µL of Wash Buffer #1 is added and the stopcock opened again. This process is repeated for the subsequent 500 µL and 250 µL Wash Buffer #2 washes as described above. The stopcock is then left open for 2 minutes to dry the spin column and the spin column is then placed into a 1.5 mL microfuge tube for the final elution as described above.

### Example 7: Component Preparation for use of the RNA isolation column

### Isolation Column Preparation:

A single layer of membrane (*e.g.,* 0.8 µm BTS) was placed on top of a polyethylene frit of about 90 µm in pore size (Porex Corp., Fairburn, GA) in the mini spin-column device (Orochem, Westmont, IL). A plastic retainer ring was placed on the assembly to secure (FIG. 5).

### Fiber Prefiltration Column Preparation:

Prefiltration columns were prepared as described in US patent application 10/631,189, the entire teaching of which is incorporated herein by reference. This is the same procedure as outlined in Example 1.

Reagent Preparation:

Reagents were prepared as in Example 1c.

### Example 8: RNA isolation from mouse tissues:

RNA Isolations from a variety of mouse tissues and cells using a membrane isolation column with and without on-column DNase digestion were performed. RNA was assayed by the RNA 6000 Nano Assay (Agilent Technologies, Palo Alto, CA, part no. 5065-4476) on the Bioanalyzer 2100 (Agilent Technologies, Palo Alto, CA, part no. G2938B) as per Manufacturer's instructions (the entire teaching of which is incorporated herein by reference).

Mouse organs that were quick frozen in liquid nitrogen immediately after harvest were obtained from Pel-Freez Biologicals (Rogers, AR). Alternatively, mouse organs can be used immediately after harvest, or preserved in a solution of RNALater (Ambion, Austin, TX).

Samples were weighed and power-homogenized in excess Lysis Solution (typically 20-fold excess) at 15,000 rpm for 30 seconds using an OMNI TH tissue homogenizer (Omni, Inc, Warrenton, VA).

Cell lines were obtained from American Type Tissue Collection (ATCC, Manassas, VA 20108) and grown according to provided instructions. Cells were trypsinized to detach them from their culture vessel, resuspended and counted with a hemocytometer. The suspension was then centrifuged at 1,000 x g for 10 minutes. The resulting pellet was resuspended in Lysis solution for a final concentration of 8.3 x 10⁶ cells/mL and vigorously vortex mixed for 1 minute. Alternatively, cells can be lysed in the cell culture vessel.

Tissue or cell homogenate (typically 300-600 µL) was added to a prefiltration column and centrifuged for 3 min at 16,000 x g in an Eppendorf 5415D microcentrifuge (Brinkman, Westbury, NY).

An equal volume of 70% ethanol was added to each of the tissue homogenates, mixed and the ethanol-containing homogenates were added to the isolation spin columns containing a single layer of 0.8 µm pore size MMM membrane (Pall Life Sciences, San Diego, CA) assembled as described in Example 7. The spin columns were then spun for at least 10 seconds at 16,000 x g. The flow-through was decanted from the collection tubes and the spin columns placed back in the collection tubes.

The spin columns were then washed twice with 500 µL wash solution#2 (above) and centrifuged for at least 10 seconds at 16,000 x g. The spin columns were then centrifuged for 2 min at 16,000 x g to remove traces of wash solution.

RNA was eluted with 25 µL nuclease-free water, centrifuging 1 min at

16,000 x g. The RNA was then stored at-70°C.

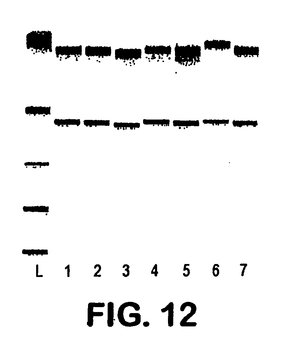

Absorbance at 260 nm and 280nm was measured on an Agilent Technologies 8453 UV/VIS spectrophotometer to confirm the presence of eluted RNA. Table 9 is a summary of the resulting RNA yields. Figure 12 is the software-generated image of the RNA 6000 Nano Assay.

**Table 9**

| Tissue | Yield by A₂₆₀ₙₘ |
|---|---|
| Brain (30 mg) | 0.8 µg/mg |
| Liver (30 mg) | 4.5 µg/mg |
| Kidney (30 mg) | 2.7 µg/mg |
| Pancreas (10 mg) | 15 µg/mg |
| Spleen (15 mg) | 4.3 µg/mg |
| HeLa (cells) (5 x 10⁶) | 15.5 µg/10⁶ |
| NIH3T3 (cells) (5 x 10⁶) | 15 µg/mg |

As can be seen in Table 9 and FIG. 12, RNA purified using the methods and devices of the present invention is of high yield, purity and integrity. In FIG. 12, lane L is the ladder for molecular weight determination, 1 is brain, 2, is kidney, 3 is liver, 4 is pancreas, 5 is spleen, 6 is HeLa, and 7 is NIH3T3.

### Example 9: Optimization of membrane

RNA was isolated from mouse spleen and thymus that were quick frozen in liquid nitrogen immediately after harvest (obtained from Pel-Freez Biologicals (Rogers, AR)) using isolation spin columns with various polymeric membranes: 0.1 µm, 0.2 µm, and 0.8 µm BTS (Pall Life Sciences, San Diego, CA), 0.8 µm MMM (Pall Life Sciences, San Diego, CA), 0.45 µm and 0.8 µm PVDF (hydrophilic polyvinylidene fluoride, Millipore,Bedford, MA). RNA was isolated as described in Example 8, however, in this Example, the type of membrane used in the spin column of the isolation step was varied. The Specific Yield (µg tcRNA/mg spleen) is shown in Table 10.

**Table 10 Yield**

| | µg tcRNA/ mg Spleen |
|---|---|
| 0.1 µm BTS | 2.6 |
| 0.2 µm BTS | 4.2 |
| 0.8 µm BTS | 5.0 |
| 0.45 µm PVDF | 1.6 |
| 0.8 µm PVDF | 1.8 |
| 0.8 µm MMM | 5.5 |

### Example 10: Comparison of genomic DNA contamination

Mouse pancreas, spleen and thymus were obtained that were quick frozen in liquid nitrogen immediately after harvest from Pel-Freez Biologicals (Rogers, AR). Spleen and thymus tissues were selected for the Examples shown herein because these are difficult tissues from which to isolate RNA due to the large amounts of gDNA present. RNA was isolated from these tissues as described in Example 9.

For samples indicated as "plus" DNase, samples were weighed and power-homogenized in excess Lysis Solution (typically 20-fold excess) at 15,000 rpm for 30 seconds using an OMNI TH tissue homogenizer (Omni, Inc, Warrenton, VA). Tissue homogenate (typically 300-600 µL) was added to a prefiltration column and centrifuged for 3 min at 16,000 x g in an Eppendorf 5415D microcentrifuge (Brinkman, Westbury, NY).

The spin columns were then washed with 350 µL wash solution #2 and centrifuged for 2 min at 16,000 x g to remove traces of wash solution.

DNase (5 units was added to 24 µL of DNase Digestion Buffer and mixed gently by pipetting. The mixture was added to the membrane surface of the column containing the partially purified RNA and incubated for 15 min at room temperature with the cap closed.

The spin columns were then washed once with 350 µL wash solution #1 and centrifuged for at least 10 seconds at 16,000 x g . The spin columns were then washed twice with 500 µL wash solution #2and centrifuged for at least 10 seconds at 16,000 x g. The spin columns were then centrifuged for 2 min at 16,000 x g to remove traces of wash solution.

RNA was eluted using 25 µL nuclease-free water, centrifuging 1 min at 16,000 x g. The RNA was then stored at -70°C.

For comparison purposes, mouse spleens were homogenized as described and processed as per manufacturer's instructions for QIAGEN RNeasy Mini Column isolation, the entire teaching of which is incorporated herein y reference. Samples were subjected to DNase digestion as per Manufacturer's instructions using QIAGEN RNase-free DNase Set, the entire teaching of which is incorporated herein y reference.

Genomic DNA contamination was quantified as described in Example 3.

Referring back to Tables 1 and 2, Table 1 (above) shows RNA yields from each of the conditions using the three tissues, and Table 2 (above) shows the levels of gDNA contamination in each, demonstrating the reduced levels of gDNA in the present invention's Standard (-DNase) RNA, and further reduction with the associated on-column DNase prototcol.

While somewhat more gDNA is removed using the combination of pre-filtration and DNase digestion, the methods and devices of the present invention alone removes substantially all of the gDNA and allows a practitioner to avoid DNase treatment if desired for a particular application.

Thus it has been shown that the methods and devices of the present invention effectively remove gDNA from a sample from which RNA is being isolated, can avoid the necessity of DNase digestion (yet are compatible with DNase digestion if necessary).

### Example 11: Additional tissue isolation

In addition to the isolation and purification procedures described above, RNA isolation from tissues high in connective tissue and contractive proteins such as skin, heart and muscle can be facilitated with Proteinase K treatment.

To begin, such tissues are homogenized, as described above, and an equal volume of water can be added to the sample. Proteinase K can then be added to a final concentration of 1 unit/100 µL, mixed and incubated at 55° C for 10 minutes. The homogenate can then be centrifuged through the pre-filter column of the present invention (with/without further processing), with or without DNase treatment as described above.

### Example 12: Purification of Labeled cRNA

Complementary RNA was prepared from total RNA isolated from mouse liver, as described in Agilent Technologies' Low RNA Input Fluorescent Linear Amplification Kit Protocol, version 1.1, pgs 9-13.

Approximately 20 µL of nuclease free-water was added to the cRNA sample bringing the total volume up to around 100 µL. To this was added approximately 350 µL of Stabilization Solution and the mixture was mixed thoroughly. To this mixture, approximately 250 µL (~96-100% purity) ethanol was added and mixed thoroughly by pipet mixing. Approximately 700 µL of the cRNA sample was transferred to a MMM column (see, FIG. 13). The sample was centrifuged for 30 seconds at 13,000 rpm. The flow-through was discarded. About 500 µL of Wash Buffer #3 was added to the column. The sample was centrifuged for 30 seconds at 13,000 rpm. The flow through was discarded. This wash step was repeated (About 500 µL of Wash Buffer #3 was added to the column. The sample was centrifuged for 30 seconds at 13,000 rpm. The flow through was discarded.) The cRNA sample was eluted using a new collection tube (~1.5 mL), 30 µL of RNase-free water was added directly to the column membrane. After 1 minute, the sample was centrifuged for 60 seconds at 13,000 rpm. The effluent was collected. This elution step was repeated again by adding 30 µL of RNase-free water to the column and proceeding as before.

Figure 14 shows five different columns used to purify cRNA, (a) 0.1 MMM, (b) 0.4 MMM, (c) 0.6 MMM, (d) 0.8 MMM, and (e) a silica-based column (a Rneasy column manufactured by Qiagen). The designation of these columns, *i.e.,* the MMM columns, are the µm pore size of the bottom surface of the asymmetric membrane column of the present invention, such that 0.4 MMM means a MMM column having a pore size of 0.4 µm. As seen from FIG. 14, the optimal pore size appears to be 0.6. Step 24 on page 13 of Agilent's Amplification Kit manual describes quantitating the cRNA on a Nanodrop instrument. Following this quantitation, the concentration of labeled cRNA was calculated by multiplying the absorbance at OD₂₆₀ by 10 (this is due to the path length of the nanodrop being 1 mm) then by 40 (simply because 1 OD unit = 40 µg/mL of RNA) then by a dilution factor (if the prep needed to be diluted to be in the linear measurement range of the Nanodrop instrument). The resulting concentration was then plotted within Excel in order to produce the graph seen in FIG. 14.

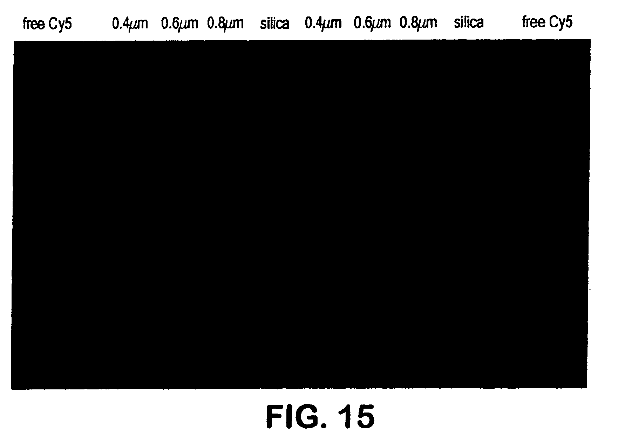

Figure 15 shows a gel image of the various cRNA preparations. The gel was generated by running 2 µL per lane of each cRNA prep (0.4, 0.6, 0.8, and silica-based) on a 1.5% agarose TBE gel for 45 minutes at 80 volts. Following electrophoresis, the gel was scanned using Amersham Typhoon 8600 variable mode imager set for fluorescence mode, at 530 volts, normal sensitivity, excitation set at 633 nm (red) and using a 670BP30 emission filter.

Example 13: Isolation of total cellular RNA from plant tissue

Various plant tissues were weighed and power-homogenized in 10 volumes of Extraction Buffer (5.5 M guanidine hydrochloride, 50 mM Bis-Tris pH 6.6, 10 mM EDTA, and 1% β-mercaptoethanol) for 30 seconds using an OMNI TH tissue homogenizer (Omni, Inc, Warrenton, VA). 400µL of tissue homogenate was transferred to a prefiltration column of the present invention and centrifuged for 3 min. at 16,000 x g in an Eppendorf 5415D microcentrifuge (Brinkman, Westbury, NY). The prefiltration column was discarded. An equal volume (400µL) of isopropyl alcohol was added to the effluent, mixed thoroughly, and transferred to an isolation column of the present invention. The spin columns were then centrifuged for 30 sec. at 16,000 x g. The effluent from each was discarded and the spin columns returned to the collection tubes.

The spin columns were washed by adding 700 µL of Wash Buffer (70%ethanol, 10mM Tris pH 8.0, 1mM EDTA) and centrifuged for 30 sec. at 16,000 x g. The wash step was repeated once with 500 µL of wash solution and extending the centrifugation time to 2 minutes. Each of the columns was then removed from its 2mL collection tube and placed into a clean nuclease-free 1.5 mL microfuge tube. RNA was eluted with 30 µL nuclease-free H₂O. RNA was stored at -20°C.

The RNA samples were diluted 5 fold in TE pH7.0 and 20 µL used for spectrophotometic analysis in a Hewlett Packard 8453 spectrophotometer. Absorbance readings were taken at 230nm (A₂₃₀), 260nm (A₂₆₀), and 280nm (A₂₈₀). The RNA concentration is equal to the absorbance at 260nm multiplied by the dilution, cuvette path length and a factor of 40ug/ml per absorbance unit.

Genomic DNA (gDNA) contamination was quantified with a 5' nuclease assay, or "real-time" PCR assay, run on the Applied Biosystems Prism 7000 Sequence Detection System (Applied Biosystems, Foster City, CA). This type of assay monitors the amount of PCR product that accumulates with every PCR cycle. This is a highly sensitive and specific assay that distinguishes between DNA and RNA.

Isolated total RNA (200 ng) from Arabidopsis leaf was added to a reaction mixture containing primers and probe specific for the 18S ribosomal RNA gene. As per manufacturer's guidelines, all samples were run in a reaction mixture consisting of both primers at 900nM, fluorescent probe at 250 nM, and 1X Taqman Universal Master Mix (Applied Biosystems, Foster City, CA part no. 43044437). Serial dilutions of Arabidopsis DNA (10 fold from 10ng to 10fg) purified from leaves with the Wizard Genomic DNA Purification Kit (Promega, Madison, WI, catalog #A1120) were used for the generation of a standard curve. All samples, standards and no-template controls were run in duplicate.

The assay amplifies a 93 base-pair fragment within an exon of the 18S ribosomal RNA gene. The primers and probe were designed using the Primer Express software package (Applied Biosystems, Foster City, CA, part no. 4329442). The primers were desalted and the probe (5' labeled with 6-FAM and 3' labeled with BHQ-1) was purified by anion exchange followed by reverse phase HPLC (Biosearch Technologies, Novato, CA). The assay cycling parameters for both assays are the default conditions set by the manufacturer, *i.e.,* 50° C for 2 min., 95° C for 10 min., then 40 cycles of 95° C for 15 sec. to 60° C for 1 min. Quantitation of gDNA in the isolated tcRNA was calculated from the Arabidopsis gDNA standard curve and those results are shown in Table 11.

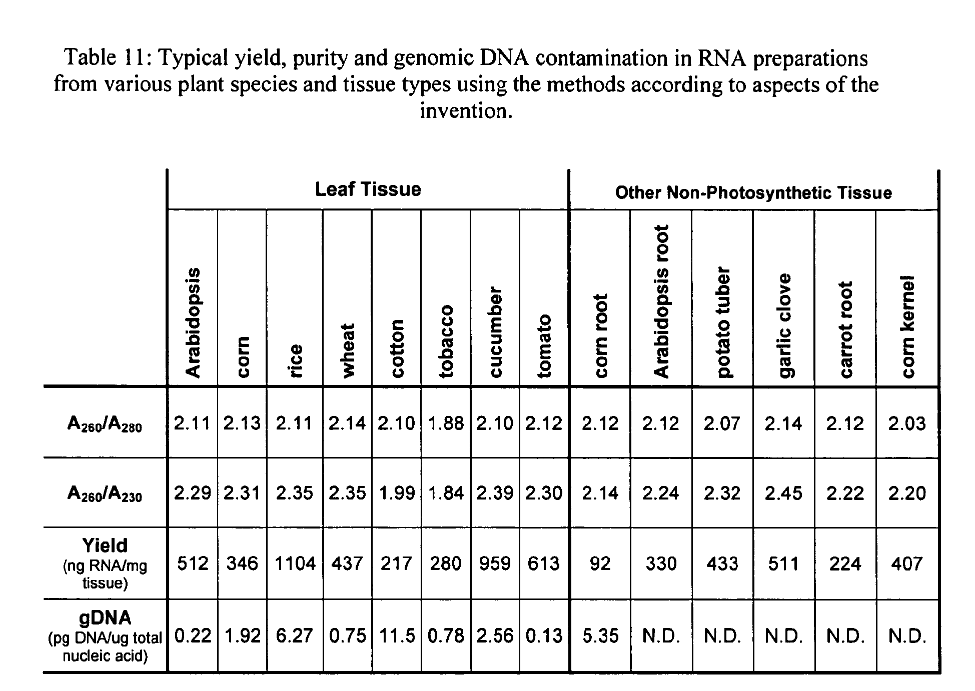

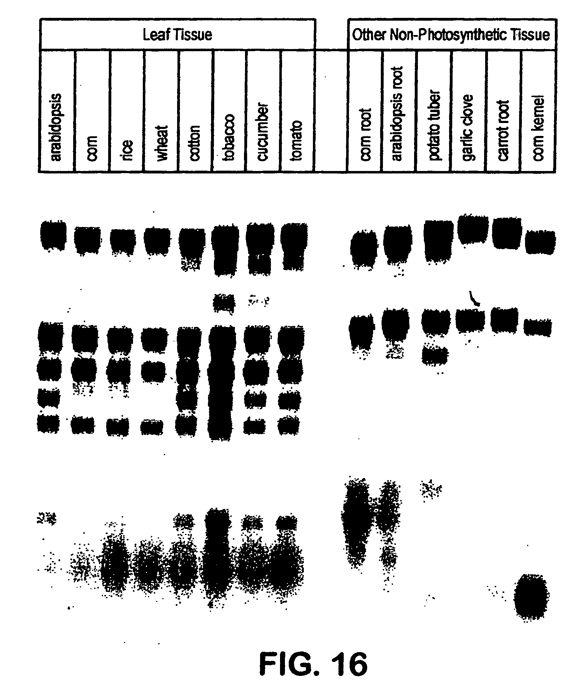

The results shown in FIG. 16 and Table 11 demonstrate the wide range of species and tissue types that can be processed with this method to isolate high quality and quantitative yields of pure RNA. The absorbance ratios A₂₆₀/A₂₈₀ and A₂₆₀/A₂₃₀ are well above 2.0, which is an indication of nucleic acid purity relative to protein and carbohydrate contamination, respectively. Genomic DNA concentrations are very low or not detectable (ND). The ribosomal bands appear crisp and distinct on a denaturing agarose gel indicating that the RNAs are intact with no evidence of degradation.

### Example 14: The efficiency of the present method in isolating RNA

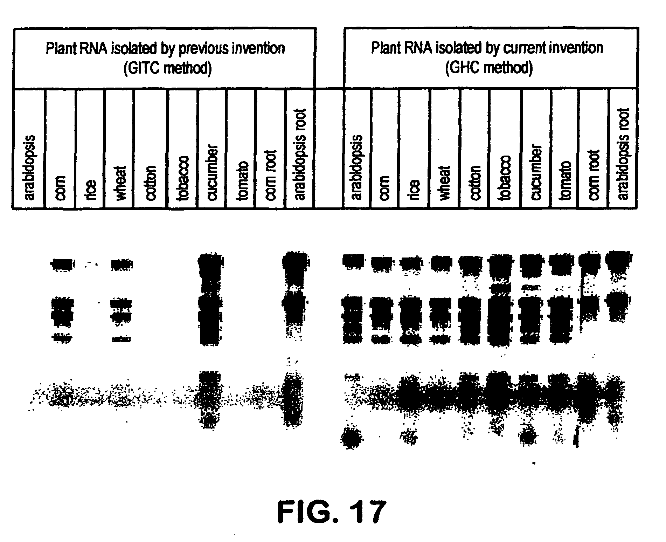

RNA was prepared from various plant species and tissue types as described in Example 13 or Example 7. The resulting purified RNA samples were analyzed by electrophoresis on a 1.2% agarose/formaldehyde denaturing gel. See FIG. 17. Five microliters of each RNA sample was denatured and run on a 1.2% agarose/formaldehyde gel as described by the manufacturer (Ambion, Austin, TX NorthernMax formaldehyde load dye (#8552), NorthernMax 10X denaturing gel buffer (#8676), and NorthernMax

### 10X running buffer (#8671)).

Although the prior (GITC) Lysis Buffer works well for some plant species and tissue types, as shown in Example 5, the current Extraction Buffer formulation and procedure yields higher quality and quantities of total RNA from a wider variety of plant species and tissue types.

### Example 15: Plant gDNA contamination is significantly less

RNA was isolated from Arabidopsis leaves using a Qiagen RLC buffer as described by the manufacturer (Qiagen, Valencia, CA Rneasy Plant Mini Kit #74903), current procedure, or current procedure without the prefiltration column. Duplicate samples of each purification method were also treated with on-column DNAase.

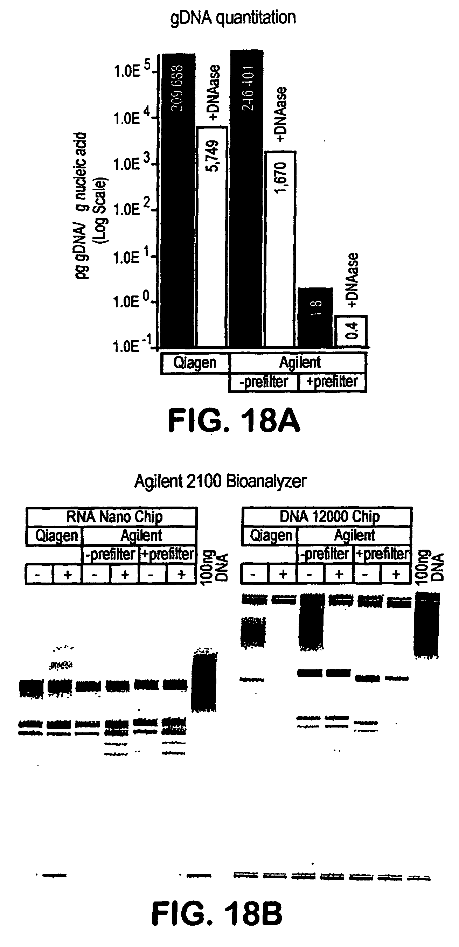

Figure 18a shows the results of real-time PCR to detect gDNA as described in Example 13. ND=not detectable. The use of the prefiltration column without any DNase treatment reduces gDNA 3-4 orders of magnitude over non-DNase treated samples prepared by Qiagen's or Agilent's protocol without a prefilter and 2-3 orders of magnitude better than the same samples DNase treated.

Figure 18b shows the visualization of genomic DNA contamination using the Agilent 2100 Bioanalyzer (Agilent Technologies, Palo Alto, CA, #G2938B): RNA 6000 Nano LabChip (#5065-4476) (left) or DNA12000 LabChip (#5064-8231) (right). One microliter of each RNA sample and 1 µL of 100ng/µL purified Arabidopsis genomic DNA were run on each chip as per manufacturers instructions. Although the samples look similar on an RNA Nano LabChip, gDNA is clearly evident in the Qiagen and Agilent - prefilter samples on the DNA 12000 Chip.

### Example 16: RNA Extraction from Plants

A plant tissue sample can be obtained from a variety of sources including plants with high resin. The present example employs White Pine needles. The tissue was prepared for homogenization. The fresh material should be small enough to enter the blades of a, for example, rotor-stator, or its equivalent. Fresh leaf material was cut into about 0.5 cm squares.

Following the preliminary tissue preparation, the tissue sample was weighed (~25 to about 100 mg) and placed in an appropriate receptacle, for example, a 15 mL round-bottom polypropylene tube works well for volumes ranging from about 1 to about 10 mL.

An extraction solution was added to the tissue preparation. Approximately 10 µL of Extraction Solution per milligram of tissue was added and then the admixture was subjected to homogenization using techniques well known to those skilled in the art. The Extraction Solution comprises approximately 1% β-mercaptoenthanol, 1% PEG3000, 5.5 M guanidine hydrochloride, 10 mM bis-tris (~pH 4-8), 10 mM EDTA. Approximately 450 µL of homogenate was processed per sample.

A prefiltration column of the present invention was employed in this method. Typically a prefiltration column can hold up to 600 mL of homogenate, however, if more than 450 mL is used, then it is advisable to filter two or more separate aliquots for prefiltration.

The tissue preparation was homogenized using, for example, a conventional rotor-stator homogenizer set at around 15,000 rpm (this is about 50% of full speed for an Omni International TH homogenizer) for about 30 seconds. These parameters can be adjusted depending on the tissue type being homogenized. (Homogenization can be effectuated by employing other methods well known to those skilled in the art such as grinding in liquid nitrogen, a dounce homogenizer, a microfuge mini-pestle, and alike.) If foaming is an issue, then to reduce foaming a practitioner can try moving the probe from side to side rather than up and down. Larger volumes (*e.g.,* greater than 10 mL) or fibrous tissues may require slightly longer homogenization times.

The homogenate is next added to a mini prefiltration column of the present invention. approximately 450 µL of homogenate (equivalent to about 45 mg of tissue) was added to a mini prefiltration column and centrifuge for about 3 minutes at full speed (approximately 16,000 x g). Wide-bore pipet tips were used to transfer since homogenate often contains particles and cell debris that plug standard tips. This prefiltration step ensures complete, or near complete, homogenization and assists in removing gDNA and other cellular contaminants. (It is advisable that mini prefiltration columns should only be used once and then discarded.) The filtrate can was then be dispensed into a collection tube.

An equal volume of isopropyl alcohol was added to the filtrate in the collection tube. (Other alcohols such as ethanol and methanol could be used for this step.) Using a device such as a pipet, the admixture was mixed and transferred to a mini isolation column. The prefiltration column removes most of the cell debris thereby eliminating essentially any pellet in the collection tube. This permits for mixing and transfer directly from the collection tube to the isolation column without transfer to a clean tube. This also eliminates potential contamination with pellet material.

The homogenate/alcohol admixture in the mini isolation column was centrifuged for about 30 seconds at around 16,000 x g. The flow-through was subsequently discarded. A new mini isolation column was used to replace the used mini isolation column in the collection tube. (If the homogenate/alcohol admixture exceeds around 700 µL, add aliquots successively into mini isolation columns, then centrifuge and discard the flow-through as described above.)

To the preparation collected in the mini isolation column, approximately 700 µL of Plant Wash Solution was added. This admixture was then centrifuged for approximately 30 seconds for about 16,000 x g. The flow-through was discarded and, in one aspect, the mini isolation column was replaced. This washing step was repeated. The Plant Wash Solution comprises around 70% ethanol, 10 mM Tris (pH ~ 8.0), and 1 mM EDTA.

Following the wash step, the mini isolation column was spun for approximately 1 minute at around 16,000 x g. (This step helps to ensure that the mini isolation column is completely dry and that no residual alcohol is carried over during elution.)

The contents of the mini isolation column was next transferred to a 1.5 mL RNase-free final collection tube. Approximately 20 - 50 µL of nuclease-free water was added. After about a minute, the preparation was centrifuged for about 1 minute at around 16,000 x g. (If more concentrated RNA samples are desired for downstream applications, then the elution volume may be decreased (to as low as 10 µL), however, if the final RNA concentration exceeds 3-5 µg/µL, quantitative recovery of the RNA may be compromised).

Figure 19(a) is a graph of the total nucleic acid yield. Figure 19(b) is a graph showing gDNA contamination which was measured by QPCR using primers/probe specific to 18S ribosomal RNA gene. Figure 19(c) represents RNA purity using the absorbance ratio A₂₆₀/A₂₈₀ which is indicative of protein contamination. Figure 19(d) represents RNA purity using absorbance ratio A₂₆₀/A₂₃₀ which indicative of carbohydrate contamination. (In FIG. 19, the terms used in this figure have the following meaning: PVP is polyvinylpyrrolidone; PVPP is polyvinylpolypyrrolidone; and PEG is polyethylene glycol.)

Figure 20 illustrates RNA scans in which the RNA was isolated from not only White Pine needles but from Milkweed, Cedar, and Spruce using the same method as described herein. Each isolation was with (+) or without (-) PEG. Clearly, in all samples examined, use of PEG in the protocol lead to a significant difference in RNA isolation. Visually this is apparent when one compares the (-) column with the (+) column. Further, the quantitative analysis reveals a similar pattern. The quantitative analysis is reflective in the row labeled "Yield."

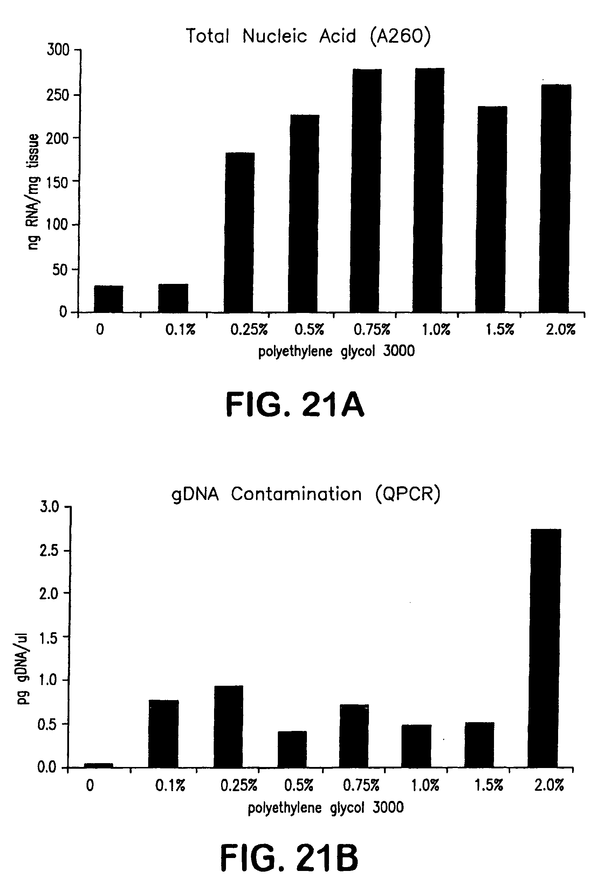

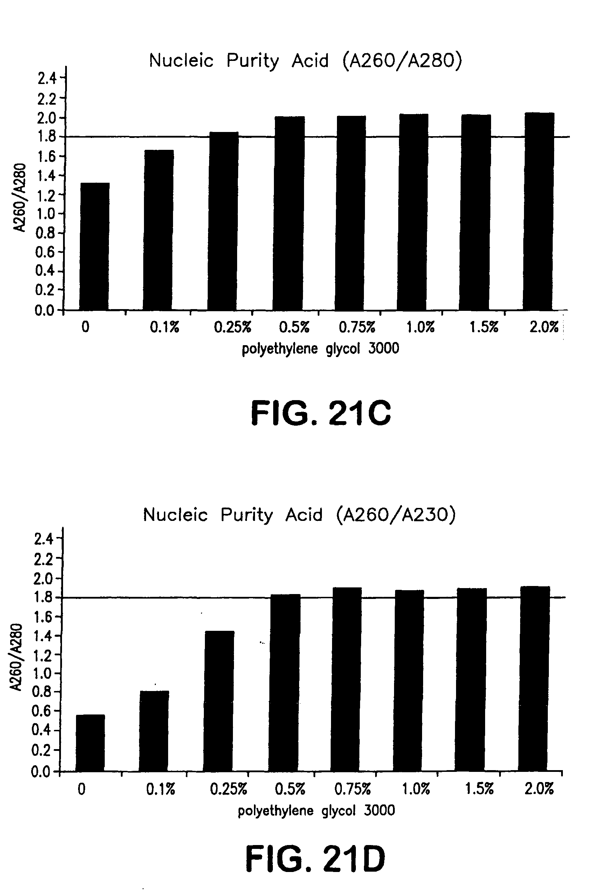

Figure 21 shows the effect of increasing concentrations of PEG on total RNA isolation using White Pine needles and the current method. Figure 21 (a) is a bar graph depicting the yield for total nucleic acid using different percentages of PEG. Under the experimental conditions, it appears that 0.75% to 1.0% PEG was optimal for yielding nucleic acid. Figure 21 (b) is a bar graph depicting genomic DNA contamination as measured by QPCR with primers/probe specific to 18S ribosomal RNA gene. Again, various percentages of PEG were employed. At 2.0% PEG one observes the highest degree of contamination, however, at the optimal concentrations of PEG (*i*.*e*., between 0.75% and 1.0%) there was minimal contamination. RNA purity was analyzed and is depicted in FIG. 2 1 (c). RNA purity was determined by absorbance, specifically, the absorbance ratio of A₂₆₀/A₂₈₀. This ratio is indicative of protein contamination (as defined by A₂₈₀). A degree of RNA purity was obtained using 0.25% to 2.0% of PEG. Figure 21 (d) illustrates RNA purity with respect to carbohydrate contamination (A₂₃₀). Significant purity is reached using 0.5% to 2.0% PEG.

While this invention has been particularly shown and described with references to specific embodiments, it will be understood by those skilled in the art that various changes in form and details may be made therein without departing from the scope of the invention as defined by the appended claims. References, patents, and patent applications cited herein are incorporated by reference in their entireties herein.

The disclosures in United States patent application Nos. 10/914,920 and 10/985,704, from which this application claims priority, and in the abstract accompanying this application are incorporated herein by reference.

## Claims

1. A method of preparing an RNA sample from a tissue comprising the following steps:
(a) forming an extraction preparation using an extraction buffer, wherein said extraction buffer comprises polyethylene glycol and a chaotropic agent;
(b) removing non-RNA components from the extraction preparation; and
(c) collecting RNA from the extraction preparation from which non-RNA components have been substantially removed.

2. The method of claim 1, wherein said non-RNA components are removed by contacting said extraction preparation with a pre-filtration column forming an RNA solution.

3. The method of claim 2, wherein said RNA solution is contacted with an RNA isolation column.

4. The method of claim 3, wherein said RNA isolation column is subjected to elution treatment resulting in the elution of RNA in substantially an isolated form.

5. The method of any preceding claim, wherein said collected RNA is from about 55% to about 65% pure.

6. The method of any preceding claim, wherein said collected RNA is from about 65% to about 75% pure.

7. The method of any preceding claim, wherein said collected RNA is from about 75% to about 85% pure.

8. The method of any preceding claim, wherein said collected RNA is from about 85% to about 95% or greater pure.

9. The method of claim 2 further comprising addition of an organic solvent to said prefiltration column, wherein said organic solvent is selected from the group consisting of isopropanol, ethanol and alike.

10. The method of claim 4, wherein said RNA isolation column is a SiCw column.

11. The method of any preceding claim further comprising homogenizing said tissue to form a homogenate prior to step (a).

12. A kit for isolating RNA comprising an extraction buffer, wherein said extraction buffer comprises polyethylene glycol and a chaotropic agent.

13. The method of claim 1 or kit of claim 12, wherein said extraction buffer comprises approximately 0.25-5% polyethylene glycol, about 0.1-2% β-mercaptoethanol, about 4-6 M guanidine hydrochloride, about 10-100 mM bis-tris pH 4-8, and about1-50 mM EDTA.

14. The method of claim 1 or kit of claim 12, wherein said chaotropic agent is selected from the group consisting of guanidine, ammonium isothiocynate, guanidine hydrochloride, and alike.

15. The kit of any of claims 12 to 14 further comprising a prefiltration column.

16. The method of claim 2 or kit of claim 15, wherein said prefiltration column includes a fiber material, wherein said fiber material has at least one layer of glass or borosilicate fiber.

17. The method or kit of claim 16, wherein said fiber material has a particle retention ranging from about 0.1 µm to about 10 µm.

18. The method or kit of claim 16 or 17, wherein said fiber material has a thickness ranging from about 50 µm to about 2000 µm.

19. The method or kit of any of claims 16 to 18, wherein said fiber material has a specific weight ranging from about 75 g/m² to about 300 g/m².

20. The kit of claim 12 further comprising an isolation column.

21. The kit of claim 20, wherein said isolation column is an RNA isolation column.

22. The method of claim 3 or kit of claim 21, wherein said RNA isolation column has a membrane formed from BTS, PVDF, nylon, nitrocellulose, polysulfone, MMM, PVP, or any composite thereof.

23. The method or kit of claim 22, wherein said RNA isolation column membrane is MMM.

24. The kit of claim 12, wherein said reagents include at least one organic solvent, nuclease free water, extraction buffer, and wash buffer.
